(19) **Europäisches Patentamt**
**European Patent Office**
**Office européen des brevets**

(11) **EP 1 978 033 A2**

(12) **EUROPEAN PATENT APPLICATION**

(43) Date of publication:
**08.10.2008 Bulletin 2008/41**

(21) Application number: **08153778.9**

(22) Date of filing: **29.04.1996**

(51) Int Cl.:
*C07K 16/00* (2006.01)     *C07K 16/22* (2006.01)
*C12N 5/16* (2006.01)      *C12N 15/13* (2006.01)
*A61K 39/00* (2006.01)

(84) Designated Contracting States:
**AT BE CH DE DK ES FI FR GB GR IE IT LI LU MC NL PT SE**

(30) Priority: **27.04.1995 US 430938**

(62) Document number(s) of the earlier application(s) in accordance with Art. 76 EPC:
**96913247.1 / 0 822 830**

(71) Applicant: **Amgen Fremont Inc.**
**Fremont, CA 94555 (US)**

(72) Inventors:
• **Kucherlapati, Raju**
**Darien, CA 06820 (US)**

• **Jakobovits, Aya**
**Menlo Park, CA 94025 (US)**
• **Klapholz, Sue**
**Stanford, CA 94305 (US)**
• **Brenner, Daniel**
**Redwood City, CA 94601 (US)**
• **Capon, Daniel, J.**
**Hillsborough, CA 94010 (US)**

(74) Representative: **Vossius & Partner**
**Siebertstrasse 4**
**81675 München (DE)**

Remarks:
This application was filed on 31-03-2008 as a divisional application to the application mentioned under INID code 62.

(54) **Human antibodies derived from immunized xenomice**

(57)     Fully human antibodies against a specific antigen can be prepared by administering the antigen to a transgenic animal which has been modified to produce such antibodies in response to antigenic challenge, but whose endogenous loci have been disabled. Various subsequent manipulations can be performed to obtain either antibodies per se or analogs thereof.

EP 1 978 033 A2

**Description**

Technical Field

The invention relates to the field of immunology, and in particular to the production of antibodies. More specifically, it concerns producing such antibodies by a process which includes the step of immunizing a transgenic animal with an antigen to which antibodies are desired. The transgenic animal has been modified so as to produce human, as opposed to endogenous, antibodies.

Background Art

PCT application WO 94/02602, published 3 February 1994 and incorporated herein by reference, describes in detail the production of transgenic nonhuman animals which are modified so as to produce fully human antibodies rather than endogenous antibodies in response to antigenic challenge. Briefly, the endogenous loci encoding the heavy and light immunoglobulin chains are incapacitated in the transgenic hosts and loci encoding human heavy and light chain proteins are inserted into the genome. In general, the animal which provides all the desired modifications is obtained by cross breeding intermediate animals containing fewer than the full complement of modifications. The preferred embodiment of nonhuman animal described in the specification is a mouse. Thus, mice, specifically, are described which, when administered immunogens, produce antibodies with human variable regions, including fully human antibodies, rather than murine antibodies that are immunospecific for these antigens.

The availability of such transgenic animals makes possible new approaches to the production of fully human antibodies. Antibodies with various immunospecificities are desirable for therapeutic and diagnostic use. Those antibodies intended for human therapeutic and in vivo diagnostic use, in particular, have been problematic because prior art sources for such antibodies resulted in immunoglobulins bearing the characteristic structures of antibodies produced by nonhuman hosts. Such antibodies tend to be immunogenic when used in humans.

The availability of the nonhuman, immunogen responsive transgenic animals described in the above-referenced WO 94/02602 make possible convenient production of human antibodies without the necessity of employing human hosts.

Disclosure of the Invention

The invention is directed to methods to produce human antibodies by a process wherein at least one step of the process includes immunizing a transgenic nonhuman animal with the desired antigen. The modified animal fails to produce endogenous antibodies, but instead produces B-cells which secrete fully human immunoglobulins. The antibodies produced can be obtained from the animal directly or from immortalized B-cells derived from the animal. Alternatively, the genes encoding the immunoglobulins with human variable regions can be recovered and expressed to obtain the antibodies directly or modified to obtain analogs of antibodies such as, for example, single chain $F_v$ molecules.

Thus, in one aspect, the invention is directed to a method to produce a fully human immunoglobulin to a specific antigen or to produce an analog of said immunoglobulin by a process which comprises immunizing a nonhuman animal with the antigen under conditions that stimulate an immune response. The nonhuman animal is characterized by being substantially incapable of producing endogenous heavy or light immunoglobulin chain, but capable of producing immunoglobulins with both human variable and constant regions. In the resulting immune response, the animal produces B cells which secrete immunoglobulins that are fully human and specific for the antigen. The human immunoglobulin of desired specificity can be directly recovered from the animal, for example, from the serum, or primary B cells can be obtained from the animal and immortalized. The immortalized B cells can be used directly as the source of human antibodies or, alternatively, the genes encoding the antibodies can be prepared from the immortalized B cells or from primary B cells of the blood or lymphoid tissue (spleen, tonsils, lymph nodes, bone marrow) of the immunized animal and expressed in recombinant hosts, with or without modifications, to produce the immunoglobulin or its analogs. In addition, the genes encoding the repertoire of immunoglobulins produced by the immunized animal can be used to generate a library of immunoglobulins to permit screening for those variable regions which provide the desired affinity. Clones from the library which have the desired characteristics can then be used as a source of nucleotide sequences encoding the desired variable regions for further manipulation to generate antibodies or analogs with these characteristics using standard recombinant techniques.

In another aspect, the invention relates to an immortalized nonhuman B cell line derived from the above described animal. In still another aspect, the invention is directed to a recombinant host cell which is modified to contain the gene encoding either the human immunoglobulin with the desired specificity, or an analog thereof which exhibits the same specificity.

In still other aspects, the invention is directed to antibodies or antibody analogs prepared by the above-described methods and to recombinant materials for their production.

In still other aspects, the invention is directed to antibodies which are immunospecific with respect to particular antigens set forth herein and to analogs which are similarly immunospecific, as well as to the recombinant materials useful to production of these antibodies.

Brief Description of the Drawings

Figure 1 is a schematic of the construction of the yH1C human heavy chain YAC.

Figure 2 is a schematic of the construction of the yK2 human kappa light chain YAC.

Figure 3 shows the serum titers of anti-IL-6 antibodies from a XenoMouse™ immunized with human IL-6 and which antibodies contain human κ light chains and/or human μ heavy chains.

Figure 4 show the serum titers of anti-TNFα antibodies from a XenoMouse™ immunized with human TNF-α and which antibodies contain human κ light chains and/or human μ heavy chains.

Figure 5 shows serum titers of anti-CD4 antibodies from a XenoMouse™ immunized with human CD4 and which antibodies contain human κ light chains and/or human μ heavy chains.

Figure 6 shows the serum titers of a XenaMouse™ immunized with 300.19 cells expressing L-selectin at their surface. In the ELISA assay used, these antibodies are detectable if they carry human μ constant region heavy chains.

Figure 7 shows the serum titers of a XenoMouse™ immunized with 300.19 cells expressing L-selectin at their surface. In the ELISA assay used, these antibodies are detectable only if they carry human κ light chains.

Figure 8 shows a FACS Analysis of human neutrophils incubated with serum from a XenoMouse™ immunized with human L-selectin and labeled with an antibody immunoreactive with human light chain κ region.

Figure 9 shows a diagram of a plasmid used to transfect mammalian cells to effect the production of the human protein gp39.

Figure 10 represents the serum titration curve of mice immunized with CHO cells expressing human gp39. The antibodies detected in this ELISA must be immunoreactive with gp39 and contain human heavy chain μ constant regions of human κ light chains.

Figure 11 is a titration curve with respect to monoclonal antibodies secreted by the hybridoma clone D5.1. This clone is obtained from a XenoMouse™ immunized with tetanus toxin C (TTC) and contains human κ light chain and human μ constant region in the heavy chain.

Figure 12 DNA sequence of the heavy chain of anti tetanus toxin monoclonal antibody D5.1.4 (a subclone of D5.1). Mutations form germline are boxed.

Figure 13 DNA sequence of the kappa light chain of anti-tetanus toxin monoclonal antibody D5.1.4. Mutations form germline are boxed.

Figure 14 shows the serum titers of anti-IL-8 antibodies of XenoMouse™ immunized with human IL-8 and which antibodies contain human κ light chains and/or human μ heavy chains.

Figure 15 Inhibition of IL-8 binding to human neutrophils by monoclonal anti-human-IL-8 antibodies.

Figure 16 (A-H) DNA sequences of the heavy chain and kappa light chain of the anti-IL-8 antibodies D1.1 (16A-B), K2.2 (16C-D), K4.2 (16E-F), and K4.3 (16G-H).

Modes of Carrying Out the Invention

In general, the methods of the invention include administering an antigen for which human forms of immunospecific reagents are desired to a transgenic nonhuman animal which has been modified genetically so as to be capable of producing human, but not endogenous, antibodies. Typically, the animal has been modified to disable the endogenous heavy and/or kappa light chain loci in its genome, so that these endogenous loci are incapable of the rearrangement required to generate genes encoding immunoglobulins in response to an antigen. In addition, the animal will have been provided, stably, in its genome, at least one human heavy chain locus and at least one human light chain locus so that in response to an administered antigen, the human loci can rearrange to provide genes encoding human variable regions immunospecific for the antigen.

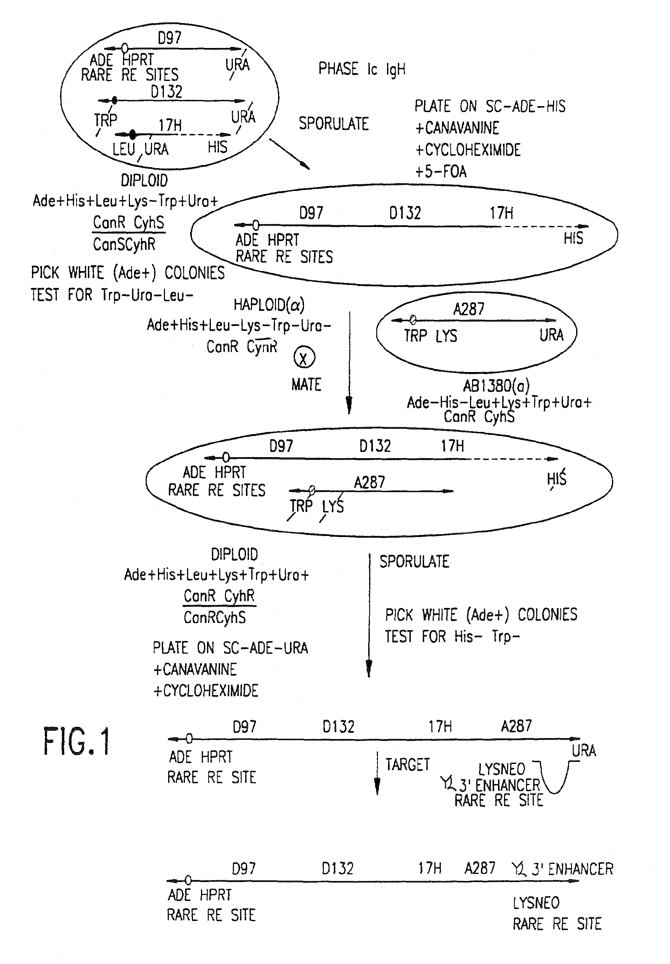

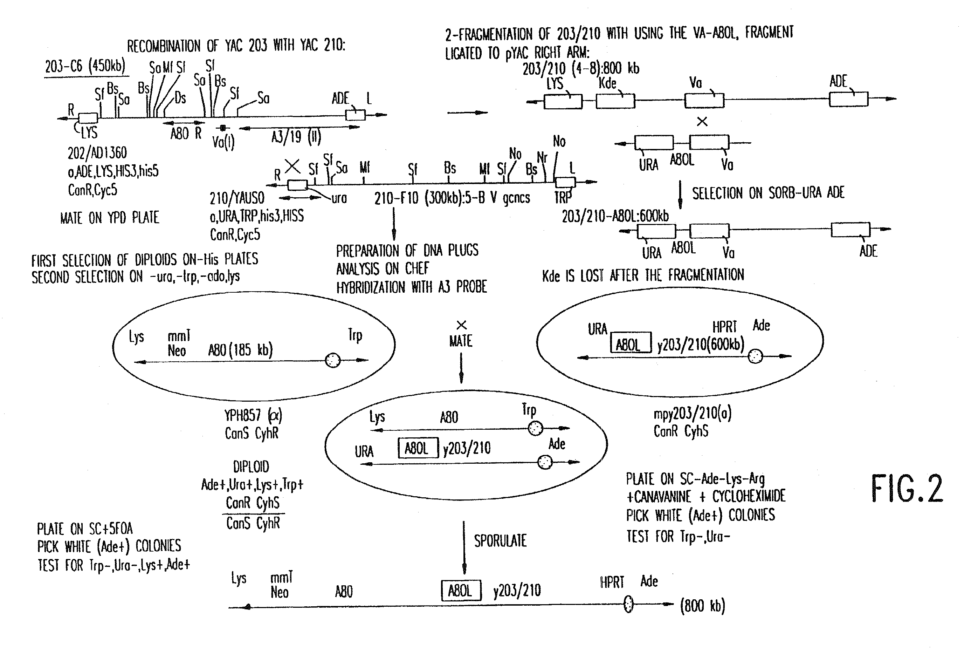

The details for constructing such an animal useful in the method of the invention are provided in the PCT application WO 94/02602 referenced above. Examples of YACs for the present invention can be found in, for example, Green et al. Nature Genetics 7:13-21 (1994). In a preferred embodiment of the XenoMouse™, the human heavy chain YAC, yH1C (1020 kb), and human light chain YAC, yK2 (880 kb) are used. yH1C is comprised of 870 kb of the human variable region, the entire D and $J_H$ region, human μ, δ, and γ2 constant regions and the mouse 3' enhancer. yK2 is comprised of 650 kb of the human kappa chain proximal variable region ($V_\kappa$), the entire $J_\kappa$ region, and $C_\kappa$ with its flanking sequences that contain the Kappa deleting element (κde). Both YACs also contain a human HPRT selectable marker on their YAC vector arm. Construction of yH1C and yK2 was accomplished by methods well known in the art. In brief, YAC clones bearing segments of the human immunoglobulin loci were identified by screening a YAC library (Calbertsen et al, PNAS 87:4256 (1990)) Overlapping clones were joined by recombination using standard techniques (Mendez et al. Genomics 26:294-307 (1995)). Details of the schemes for assembling yH1C and yK2 are shown in Figure 1 and Figure 2 respectively.

yK2 was constructed from the clones A80-C7, A210-F10 and A203-C6 from the Olson library, disclosed in, for example, Burke et al., Science 236:806-812 (1987), Brownstein et al., Science 244:1348-1351 (1989), and Burke et al., Methods in Enzymology 194:251-270 (1991).

For production of the desired antibodies, the first step is administration of the antigen. Techniques for such administration are conventional and involve suitable immunization protocols and formulations which will depend on the nature of the antigen per se. It may be necessary to provide the antigen with a carrier to enhance its immunogenicity and/or to include formulations which contain adjuvants and/or to administer multiple injections and/or to vary the route of the immunization, and the like. Such techniques are standard and optimization of them will depend on the characteristics of the particular antigen for which immunospecific reagents are desired.

As used herein, the term "immunospecific reagents" includes immunoglobulins and their analogs. The term "analogs" has a specific meaning in this context. It refers to moieties that contain the fully human portions of the immunoglobulin which account for its immunospecificity. In particular, complementarity determining regions (CDRs) are required, along with sufficient portions of the framework (Frs) to result in the appropriate three dimensional conformation. Typical immunospecific analogs of antibodies include $F(ab")_2$, Fab', and Fab regions. Modified forms of the variable regions to obtain, for example, single chain $F_v$ analogs with the appropriate immunospecificity are known. A review of such $F_v$ construction is found, for example, in Huston et al., Methods in Enzymology 203:46-63 (1991). The construction of antibody analogs with multiple immunospecificities is also possible by coupling the variable regions from one antibody to those of second antibody.

The variable regions with fully human characteristics can also be coupled to a variety of additional substances which can provide toxicity, biological functionality, alternative binding specificities and the like. The moieties including the fully human variable regions produced by the methods of the invention include single-chain fusion proteins, molecules coupled by covalent methods other than those involving peptide linkages, and aggregated molecules. Examples of analogs which include variable regions coupled to additional molecules covalently or noncovalently include those in the following nonlimiting illustrative list. Traunecker, A. et al. Int. J. Cancer Supp (1992) Supp 7:51-52 describe the bispecific reagent janusin in which the $F_v$ region directed to CD3 is coupled to soluble CD4 or to other ligands such as OVCA and IL-7. Similarly, the fully human variable regions produced by the method of the invention can be constructed into $F_v$ molecules and coupled to alternative ligands such as those illustrated in the cited article. Higgins, P.J. et al J.Infect Disease (1992) 166:198-202 described a heteroconjugate antibody composed of OKT3 cross-linked to an antibody directed to a specific sequence in the V3 region of GP120. Such heteroconjugate antibodies can also be constructed using at least the human variable regions contained in the immunoglobulins produced by the invention methods. Additional examples of bispecific antibodies include those described by Fanger, M.W. et al. Cancer Treat Res (1993) 68:181-194 and by Fanger, M.W. et al. Crit Rev Immunol (1992) 12:101-124. Conjugates that are immunotoxins including conventional antibodies have been widely described in the art. The toxins may be coupled to the antibodies by conventional coupling techniques or immunotoxins containing protein toxin portions can be produced as fusion proteins. The analogs of the present invention can be used in a corresponding way to obtain such immunotoxins. Illustrative of such immunotoxins are those described by Byers, B.S. et al. Seminars Cell Biol (1991) 2:59-70 and by Fanger, M.W. et al. Immunol Today (1991) 12:51-54.

It will also be noted that some of the immunoglobulins and analogs of the invention will have agonist activity with respect to antigens for which they are immunospecific in the cases wherein the antigens perform signal transducing functions. Thus, a subset of antibodies or analogs prepared according to the methods of the invention which are immunospecific for, for example, a cell surface receptor, will be capable of eliciting a response from cells bearing this receptor corresponding to that elicited by the native ligand. Furthermore, antibodies or analogs which are immunospecific for substances mimicking transition states of chemical reactions will have catalytic activity. Hence, a subset of the antibodies and analogs of the invention will function as catalytic antibodies.

In short, the genes encoding the immunoglobulins produced by the transgenic animals of the invention can be retrieved and the nucleotide sequences encoding the fully human variable region can be manipulated according to known techniques to provide a variety of analogs such as those described above. In addition, the immunoglobulins themselves containing the human variable regions can be modified using standard coupling techniques to provide conjugates retaining immunospecific regions.

Thus, immunoglobulin "analogs" refers to the moieties which contain those portions of the antibodies of the invention which retain their human characteristics and their immunospecificity. These will retain sufficient human variable regions to provide the desired specificity.

As stated above, all of the methods of the invention include administering the appropriate antigen to the transgenic animal. The recovery or production of the antibodies themselves can be achieved in various ways.

First, and most straightforward, the polyclonal antibodies produced by the animal and secreted into the bloodstream can be recovered using known techniques. Purified forms of these antibodies can, of course, be readily prepared by standard purification techniques, preferably including affinity chromatography with Protein A, anti-immunoglobulin, or the antigen itself. In any case, in order to monitor the success of immunization, the antibody levels with respect to the antigen in serum will be monitored using standard techniques such as ELISA, RIA and the like.

For some applications only the variable regions of the antibodies are required. Treating the polyclonal antiserum with suitable reagents so as to generate Fab', Fab, or $F(ab")_2$ portions results in compositions retaining fully human characteristics. Such fragments are sufficient for use, for example, in immunodiagnostic procedures involving coupling the immunospecific portions of immunoglobulins to detecting reagents such as radioisotopes.

Alternatively, immunoglobulins and analogs with desired characteristics can be generated from immortalized B cells derived from the transgenic animals used in the method of the invention or from the rearranged genes provided by these animals in response to immunization.

Thus, as an alternative to harvesting the antibodies directly from the animal, the B cells can be obtained, typically from the spleen, but also, if desired, from the peripheral blood lymphocytes or lymph nodes and immortalized using any of a variety of techniques, most commonly using the fusion methods described by Kohler and Milstein Nature 245:495 (1975) The resulting hybridomas (or otherwise immortalized B cells) can then be cultured as single colonies and screened for secretion of antibodies of the desired specificity. As described above, the screen can also include a confirmation of the fully human character of the antibody. For example, as described in the examples below, a sandwich ELISA wherein the monoclonal in the hybridoma supernatant is bound both to antigen and to an antihuman constant region can be employed. After the appropriate hybridomas are selected, the desired antibodies can be recovered, again using conventional techniques. They can be prepared in quantity by culturing the immortalized B cells using conventional methods, either in vitro or in vivo to produce ascites fluid. Purification of the resulting monoclonal antibody preparations is less burdensome that in the case of serum since each immortalized colony will secrete only a single type of antibody. In any event, standard purification techniques to isolate the antibody from other proteins in the culture medium can be employed.

As an alternative to obtaining human immunoglobulins directly from the culture of immortalized B cells derived from the animal, the immortalized cells can be used as a source of rearranged heavy chain and light chain loci for subsequent expression and/or genetic manipulation. Rearranged antibody genes can be reverse transcribed from appropriate mRNAs to produce cDNA. If desired, the heavy chain constant region can be exchanged for that of a different isotype or eliminated altogether. The variable regions can be linked to encode single chain $F_v$ regions. Multiple $F_v$ regions can be linked to confer binding ability to more than one target or chimeric heavy and light chain combinations can be employed. Once the genetic material is available, design of analogs as described above which retain both their ability to bind the desired target, and their human characteristics, is straightforward.

Once the appropriate genetic material is obtained and, if desired, modified to encode an analog, the coding sequences, including those that encode, at a minimum, the variable regions of the human heavy and light chain, can be inserted into expression systems contained on vectors which can be transfected into standard recombinant host cells. As described below, a variety of such host cells may be used; for efficient processing, however, mammalian cells are preferred. Typical mammalian cell lines useful for this purpose include CHO cells, 293 cells, or NSO cells.

The production of the antibody or analog is then undertaken by culturing the modified recombinant host under culture conditions appropriate for the growth of the host cells and the expression of the coding sequences. The antibodies are then recovered from the culture. The expression systems are preferably designed to include signal peptides so that the resulting antibodies are secreted into the medium; however, intracellular production is also possible.

In addition to deliberate design of modified forms of the immunoglobulin genes to produce analogs, advantage can be taken of phage display techniques to provide libraries containing a repertoire of antibodies with varying affinities for the desired antigen. For production of such repertoires, it is unnecessary to immortalize the B cells from the immunized animal; rather, the primary B cells can be used directly as a source of DNA. The mixture of cDNAs obtained from B cells, e.g., derived from spleens, is used to prepare an expression library, for example, a phage display library transfected into E. coli. The resulting cells are tested for immunoreactivity to the desired antigen. Techniques for the identification of high affinity human antibodies from such libraries are described by Griffiths, A.D., et al., EMBO J (1994) 13:3245-3260; by Nissim, A., et al. ibid, 692-698, and by Griffiths, A.D., et al., ibid, 12:725-734. Ultimately, clones from the library are identified which produce binding affinities of a desired magnitude for the antigen, and the DNA encoding the product responsible for such binding is recovered and manipulated for standard recombinant expression. Phage display libraries may also be constructed using previously manipulated nucleotide sequences and screened in similar fashion. In general, the cDNAs encoding heavy and light chain are independently supplied or are linked to form $F_v$ analogs for production in the phage library.

The phage library is then screened for the antibodies with highest affinity for the antigen and the genetic material recovered from the appropriate clone. Further rounds of screening can increase the affinity of the original antibody isolated. The manipulations described above for recombinant production of the antibody or modification to form a desired analog can then be employed.

Combination of phage display technology with the XenoMouse™ offers a significant advantage over previous applications of phage display. Typically, to generate a highly human antibody by phage display, a combinatorial antibody library is prepared either from human bone marrow or from peripheral blood lymphocytes as described by Burton, D.R., et al., Proc. Natl. Acad. Sci. USA (1991) 88:10134-10137. Using this approach, it has been possible to isolate high affinity antibodies to human pathogens from infected individuals, i.e. from individuals who have been "immunized" as described in Burton, D.R., et al., Proc. Natl. Acad. Sci. USA (1991) 88:10134-10137, Zebedee, S.L., et al. Proc. Natl. Acad. Sci. USA (1992) 89:3175-3179, and Barbas III, C.F., et al., Proc. Natl. Acad. Sci. USA (1991) 89:10164-20168. However, to generate antibodies reactive with human antigens, it has been necessary to generate synthetic libraries (Barbas III C.F., et al., Proc. Natl. Acad. Sci. USA (1991) 89:4457-4461, Crameri, A. et al., BioTechniques (1995) 88: 194-196) or to prepare libraries from either autoimmune patients (Rapoport, B., et al., Immunol. Today (1995) 16:43-49, Portolano, S., et al., J. Immunol. (1993) 151:2839-2851, and Vogel, M., et al., Eur J. Immunol. (1994) 24:1200-1207) or normal individuals, i.e. naive libraries (Griffiths, A.D., et al., EMBO J. (1994) 13:3245-3260, Griffiths, A.D., et al., EMBO J. (1993) 12:725-734, Persson, M.A.A., et al., Proc. Natl. Acad. Sci. USA (1991) 88:2432-2436, Griffiths, A.D., Curr. Opin. Immunol. (1993) 5:263-267, Hoogenboom, H.R., et al., J. Mol. Biol. (1992) 227:381-388, Lerner, R.A., et al., Science (1992) 258:1313-1314, and Nissim A., et al., EMBO J. (1994) 13:692-698. Typically, high affinity antibodies to human proteins have proven very difficult to isolate in this way. As is well known, affinity maturation requires somatic mutation and somatic mutation, in turn, is antigen driven. In the XenoMouse, repeated immunization with human proteins will lead to somatic mutation and, consequently, high affinity antibodies. The genes encoding these antibodies can be readily amplified by PCR as described in Marks, J.D., et al., J. Mol. Biol. (1991) 581-596 and immunospecific antibodies isolated by standard panning techniques, Winter, G., et al., Annu. Rev. Immunol. (1994) 12:433-55 and Barbas III, C.F., et al., Proc. Natl. Acad. Sci. USA (1991) 88:7978-7982.

As above, the modified or unmodified rearranged loci are manipulated using standard recombinant techniques by constructing expression systems operable in a desired host cell, such as, typically, a Chinese hamster ovary cell, and the desired immunoglobulin or analog is produced using standard recombinant expression techniques, and recovered and purified using conventional methods.

The application of the foregoing processes to antibody production has enabled the preparation of human immunospecific reagents with respect to antigens for which human antibodies have not heretofore been available. The immunoglobulins that result from the above-described methods and the analogs made possible thereby provide novel compositions for use in analysis, diagnosis, research, and therapy. The particular use will, of course, depend on the immunoglobulin or analog prepared. In general, the compositions of the invention will have utilities similar to those ascribable to nonhuman antibodies directed against the same antigen. Such utilities include, for example, use as affinity ligands for purification, as reagents in immunoassays, as components of immunoconjugates, and as therapeutic agents for appropriate indications.

Particularly in the case of therapeutic agents or diagnostic agents for use in vivo, it is highly advantageous to employ antibodies or their analogs with fully human characteristics. These reagents avoid the undesired immune responses engendered by antibodies or analogs which have characteristics marking them as originating from nonhuman species. Other attempts to "humanize" antibodies do not result in reagents with fully human characteristics. For example, chimeric antibodies with murine variable regions and human constant regions are easily prepared, but, of course, retain murine characteristics in the variable regions. Even the much more difficult procedure of "humanizing" the variable regions by manipulating the genes encoding the amino acid sequences that form the framework regions does not provide the desired result since the CDRs, typically of nonhuman origin, cannot be manipulated without destroying immunospecificity.

Thus, the methods of the present invention provide, for the first time, immunoglobulins that are fully human or analogs which contain immunospecific regions with fully human characteristics.

There are large numbers of antigens for which human antibodies and their human analogs would be made available by the methods of the invention. These include, but are not limited to, the following nonlimiting set:

leukocyte markers, such as CD2, CD3, CD4, CD5, CD6, CD7, CD8, CD11a,b,c, CD13, CD14, CD18, CD19, CD20, CD22, CD23, CD27 and its ligand, CD28 and its ligands B7.1, B7.2, B7.3, CD29 and its ligand, CD30 and its ligand, CD40 and its ligand gp39, CD44, CD45 and isoforms, Cdw52 (Campath antigen), CD56, CD58, CD69, CD72, CTLA-4, LFA-1 and TCR

histocompatibility antigens, such as MHC class I or II, the Lewis Y antigens, Slex, Sley, Slea, and Selb;

adhesion molecules, including the integrins, such as VLA-1, VLA-2, VLA-3, VLA-4, VLA-5, VLA-6, LFA-1, Mac-1, $\alpha V\beta 3$, and p150,95; and

the selectins, such as L-selectin, E-selectin, and P-selectin and their counterreceptors VCAM-1, ICAM-1, ICAM-2, and LFA-3;

interleukins, such as IL-1, IL-2, IL-3, IL-4, IL-5, IL-6, IL-7, IL-8, IL-9, IL-10, IL-11, IL-12, IL-13, IL-14, and IL-15;

interleukin receptors, such as IL-1R, IL-2R, IL-3R, IL-4R, IL-5R, IL-6R, IL-7R, IL-8R, IL-9R, IL-10R, IL-11R, IL-12R, IL-13R, IL-14R and IL-15R;

chemokines, such as PF4, RANTES, MIP1$\alpha$, MCP1, IP-10, ENA-78, NAP-2, Gro$\alpha$, Gro$\beta$, and IL-8;

growth factors, such as TNFalpha, TGFbeta, TSH, VEGF/VPF, PTHrP, EGF family, FGF, PDGF family, endothelin, Fibrosin ($F_sF_{-1}$), Laminin, and gastrin releasing peptide (GRP);

growth factor receptors, such as TNFalphaR, RGFbetaR, TSHR, VEGFR/VPFR, FGFR, EGFR, PTHrPR, PDGFR family, EPO-R, GCSF-R and other hematopoietic receptors;

interferon receptors, such as IFN$\alpha$R, IFN$\beta$R, and IFN$_\gamma$R;

Igs and their receptors, such as IGE, FceRI, and FceRII;

tumor antigens, such as her2-neu, mucin, CEA and endosialin;

allergens, such as house dust mite antigen, lol p1 (grass) antigens, and urushiol;

viral proteins, such as CMV glycoproteins B, H, and gCIII, HIV-1 envelope glycoproteins, RSV envelope glycoproteins, HSV envelope glycoproteins, EBV envelope glycoproteins, VZV, envelope glycoproteins, HPV envelope glycoproteins, Hepatitis family surface antigens;

toxins, such as pseudomonas endotoxin and osteopontin/uropontin, snake venom, spider venom, and bee venom;

blood factors, such as complement C3b, complement C5a, complement C5b-9, Rh factor, fibrinogen, fibrin, and myelin associated growth inhibitor;

enzymes, such as cholesterol ester transfer protein, membrane bound matrix metalloproteases, and glutamic acid decarboxylase (GAD); and

miscellaneous antigens including ganglioside GD3, ganglioside GM2, LMP1, LMP2, eosinophil major basic protein, PTHrp, eosinophil cationic protein, pANCA, Amadori protein, Type IV collagen, glycated lipids, v-interferon, A7, P-glycoprotein and Fas (AFO-1) and oxidized-LDL.

Particularly preferred immunoglobulins and analogs are those immunospecific with respect to human IL-6, human IL-8, human TNF$\alpha$, human CD4, human L-selectin, human PTHrp and human gp39. Antibodies and analogs immunoreactive with human TNF$\alpha$ and human IL-6 are useful in treating cachexia and septic shock as well as autoimmune disease. Antibodies and analogs immunoreactive with GP39 or with L-selectin are also effective in treating or preventing autoimmune disease. In addition, anti-gp39 is helpful in treating graft versus host disease, in preventing organ transplant rejection, and in treating glomerulonephritis. Antibodies and analogs against L-selectin are useful in treating ischemia associated with reperfusion injury. Antibodies to PTHrp are useful in treating bone disease and metastatic cancer. In a particular embodiment, human antibodies against IL-8 may be used for the treatment or prevention of a pathology or condition associated with IL-8. Such conditions include, but are not limited to, tumor metastasis, reperfusion injury, pulmonary edema, asthma, ischemic disease such as myocardial infarction,inflammatory bowel disease (such as Crohn's disease and ulcerative colitis), encephalitis, uveitis, autoimmune diseases (such as rheumatoid arthritis, Sjögren's syndrome, vasculitis), osteoarthritis, gouty arthritis, nephritis, renal failure, dermatological conditions such as inflammatory dermatitis, psoriasis, vasculitic urticaria and allergic angiitis, retinal uveitis, conjunctivitis, neurological disorders such as stroke, multiple sclerosis and meningitis, acute lung injury, adult respiratory distress syndrome (ARDS), septic shock, bacterial pneumonia, diseases involving leukocyte diapedesis, CNS inflammatory disorder, multiple organ failure, alcoholic hepatitis, antigen-antibody complex mediated diseases, inflammation of the lung (such as pleurisy, aveolitis, vasculitis, pneumonia, chronic bronchitis, bronchiectasis, cystic fibrosis), Behcet disease, Wegener's granulomatosis, and vasculitic syndrome.

Typical autoimmune diseases which can be treated using the above-mentioned antibodies and analogs include systemic lupus erythematosus, rheumatoid arthritis, psoriasis, Sjogren's scleroderma, mixed connective tissue disease, dermatomyositis, polymyositis, Reiter's syndrome, Behcet's disease, Type 1 diabetes, Hashimoto's thyroiditis, Grave's disease, multiple sclerosis, myasthenia gravis and pemphigus.

For therapeutic applications, the antibodies may be administered in a pharmaceutically acceptable dosage form. They may be administered by any means that enables the active agent to reach the desired site of action, for example, intravenously as by bolus or by continuous infusion over a period of time, by intramuscular, subcutaneous, intraarticular, intrasynovial, intrathecal, oral, topical or inhalation routes. The antibodies may be administered as a single dose or a series of treatments.

For parenteral administration, the antibodies may be formulated as a solution, suspension, emulsion or lyophilized powder in association with a pharmaceutically acceptable parenteral vehicle. If the antibody is suitable for oral administration, the formulation may contain suitable additives such as, for example, starch, cellulose, silica, various sugars, magnesium carbonate, or calcium phosphate. Suitable vehicles are described in the most recent edition of Remington's Pharmaceutical Sciences, A. Osol, a standard reference text in this field.

For prevention or treatment of disease, the appropriate dosage of antibody will depend upon known factors such as the pharmacodynamic characteristics of the particular antibody, its mode and route of administration, the age, weight, and health of the recipient, the type of condition to be treated and the severity and course of the condition, frequency of treatment, concurrent treatment and the physiological effect desired. The examples below are intended to illustrate but not to limit the invention.

In these examples, mice, designated XenoMouse™, are used for initial immunizations. A detailed description of the XenoMouse™ is found in the above referenced PCT application WO 94/02602. Immunization protocols appropriate to each antigen are described in the specific examples below. The sera of the immunized XenoMouse™ (or the supernatants from immortalized B cells) were titrated for antigen specific human antibodies in each case using a standard ELISA format. In this format, the antigen used for immunization was immobilized onto wells of microtiter plates. The plates were washed and blocked and the sera (or supernatants) were added as serial dilutions for 1-2 hours of incubation. After washing, bound antibody having human characteristics was detected by adding antihuman $\kappa$, $\mu$, or $\gamma$ chain antibody conjugated to horseradish peroxidase (HRP) for one hour. After again washing, the chromogenic reagent o-phenylene diamine (OPD) substrate and hydrogen peroxide were added and the plates were read 30 minutes later at 492 nm using a microplate reader.

Unless otherwise noted, the antigen was coated using plate coating buffer (0.1 M carbonate buffer, pH 9.6); the assay blocking buffer used was 0.5% BSA, 0.1% Tween 20 and 0.01% thimerosal in PBS; the substrate buffer used in color development was citric acid 7.14 g/l; dibasic sodium phosphate 17.96 g/l; the developing solution (made immediately before use) was 10 ml substrate buffer; 10 mg OPD, plus 5 ml hydrogen peroxide; the stop solution (used to stop color development) was 2 M sulfuric acid. The wash solution was 0.05% Tween 20 in PBS.

<u>Example 1</u>

<u>Human Antibodies Against Human IL-6</u>

Three to five XenoMouse™ aged 8-20 weeks were age-matched and immunized intraperitoneally with 50 $\mu$g human IL-6 emulsified in incomplete Freund's adjuvant for primary immunization and in complete Freund's adjuvant for subsequent injections. The mice received 6 injections 2-3 weeks apart. Serum titers were determined after the second dose and following each dose thereafter. Bleeds were performed from the retrobulbar plexus 6-7 days after injections. The blood was allowed to clot at room temperature for about 2 hours and then incubated at 4°C for at least 2 hours before separating and collecting the sera.

ELISAs were conducted as described above by applying 100 $\mu$l/well of recombinant human IL-6 at 2 $\mu$g/ml in coating buffer. Plates were then incubated at 4°C overnight or at 37°C for 2 hours and then washed three times in washing buffer. Addition of 100 $\mu$l/well blocking buffer was followed by incubation at room temperature for 2 hours, and an additional 3 washes.

Then, 50 $\mu$l/well of diluted serum samples (and positive and negative controls) were added to the plates. Plates were then incubated at room temperature for 2 hours and again washed 3 times.

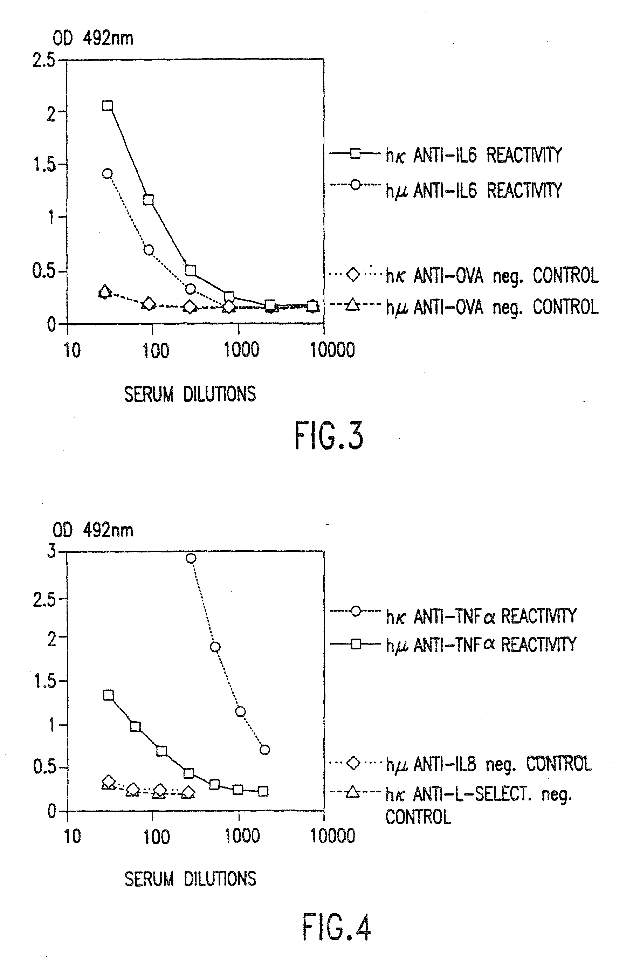

After washing, 100 $\mu$l/well of either mouse antihuman $\mu$ chain antibody conjugated to HRP at 1/2,000 or mouse antihuman $\kappa$ chain antibody conjugated to HRP at 1/2,000, diluted in blocking buffer was added. After a 1 hour incubation at room temperature, the plates were washed 3 times and developed with OPD substrate for 10-25 minutes. 50 $\mu$l/well of stop solution was then added and the results read on an ELISA plate reader at 492 nm. The dilution curves resulting from the titration of serum from XenoMouse™ after 6 injections are shown in Figure 3. The data in Figure 3 show production of anti-IL-6 immunoreactive with antihuman $\kappa$ and antihuman $\mu$ detectable at serum dilutions above 1:1,000.

Example 2

Human Antibodies Against Human TNFα

Immunization and serum preparation were conducted as described in Example 1 except that human recombinant TNFα (at 5μg per injection) was substituted for human IL-6. ELISAs were conducted as described in Example 1 except that the initial coating of the ELISA plate employed 100 μl/well recombinant human TNFα at 1 μg/ml in coating buffer.

The dilution curves for serum from XenoMouse™ after 6 inductions obtained are shown in Figure 4. Again significant titers of human anti-TNFα binding were shown.

Serum titers for hγ, hμ, and hκ after one and two immunizations of the XenoMouse™ are shown in Table 1. When challenged with TNF-α, the XenoMouse™ switches isotypes from a predominant IgM response in the first immunization to an immune response with a large IgG component in the second immunization.

TABLE 2. <u>Anti TNF-alpha serum titer responses of Xenomouse-2</u>.

| Bleed 1: after 2 immunizations Bleed 2: after 3 immunizations | | | | |
|---|---|---|---|---|
| | | **ELISA Serum titers Specific for TNF-alpha** | | |
| | **XM2** | **titer (via hγ)** | **titer (via hμ)** | **titer (via hκ)** |
| 1 | bleed 1 | 500 | 3,000 | 1,500 |
| | bleed 2 | 10,000 | 8,000 | 15,000 |
| 2 | bleed 1 | 200 | 3,000 | 500 |
| | bleed 2 | 2,700 | 5,000 | 1,000 |
| 3 | bleed 1 | <500 | 2,000 | 1,500 |
| | bleed 2 | 15,000 | 24,000 | 25,000 |
| 4 | bleed 1 | 500 | 2,500 | 1,500 |
| | bleed 2 | 70,000 | 4,000 | 72,000 |
| 5 | bleed 1 | <500 | 2,500 | 1,500 |
| | bleed 2 | 1,000 | 10,000 | 7,000 |
| 6 | bleed 1 | 1,000 | 13,000 | 4,500 |
| | bleed 2 | 10,000 | 24,000 | 25,000 |
| 7 | bleed 1 | <500 | 2,500 | 1,500 |
| | bleed 2 | 5,000 | 4,000 | 9,000 |
| 8 | bleed 1 | <500 | 1,000 | 500 |
| | bleed 2 | 2,700 | 5,000 | 9,000 |
| 9 | bleed 1 | 200 | 6,000 | 4,000 |
| | bleed 2 | 40,000 | 80,000 | 80,000 |
| 10 | bleed 1 | 200 | 2,000 | 500 |
| | bleed 2 | 15,000 | 8,000 | 60,000 |
| 11 | bleed 1 | 1,500 | 1,000 | 1,500 |
| | bleed 2 | 24,000 | 2,700 | 72,000 |
| 12 | bleed 1 | 200 | 2,000 | 1,000 |
| | bleed 2 | 10,000 | 4,000 | 25,000 |
| 13 | bleed 1 | 500 | 30,000 | 500 |
| | bleed 2 | 2,000 | 4,000 | 12,000 |

Example 3

Human antibodies Against Human CD4

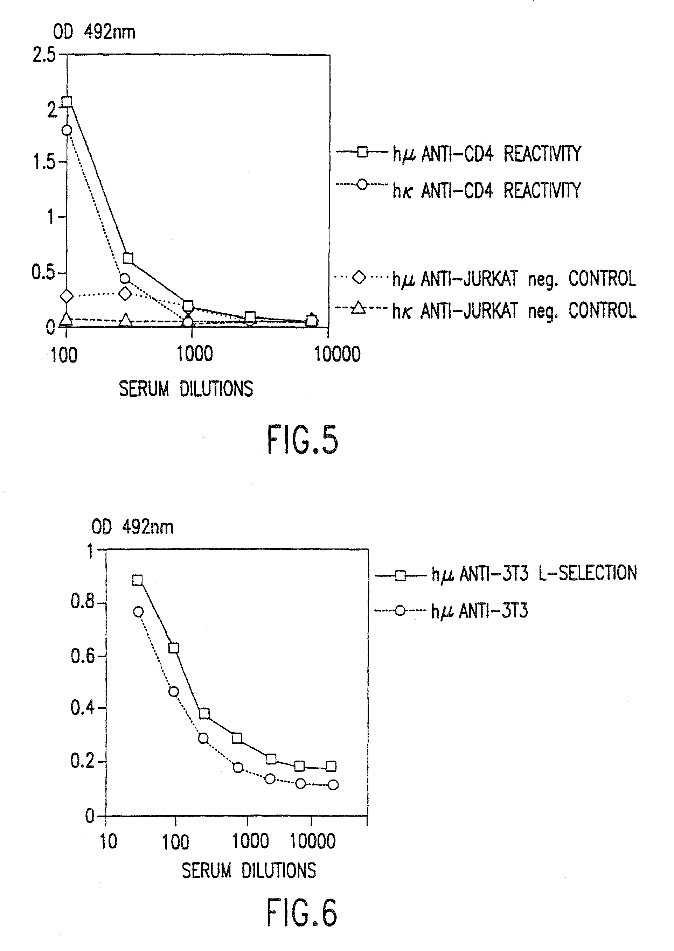

The human CD4 antigen was prepared as a surface protein using human CD4$\zeta$ on transfected recombinant cells as follows. Human CD4$\zeta$ consists of the extracellular domain of CD4, the transmembrane domain of CD4, and the cytoplasmic domain corresponding to residues 31-142, of the mature $\zeta$ chain of the CD3 complex. Human CD4 zeta (F15 LTR) as described in Roberts et al., Blood (1994) 84:2878 was introduced into the rat basophil leukemic cell line RBL-2H3, described by Callan, M., et al., Proc Natl Acad Sci USA (1993) 90:10454 using the Kat high efficiency transduction described by Finer et al., Blood (1994) 83:43. Briefly, RBL-2H3 cells at $10^6$ cells per well were cultured in 750 $\mu$l DMEM$^{low}$ + 20% FBS (Gibco) and 16 $\mu$g/ml polybrene with an equal volume of proviral supernatant for 2 hours at 37˚C, 5% CO$_2$. One ml of medium was removed and 750 $\mu$l of infection medium and retroviral supernatant were added to each well and the cultures incubated overnight. The cells were washed and expanded in DMEM$^{low}$ + 10% FBS until sufficient cells were available for sorting. The CD4 zeta transduced RBL-2H3 cells were sorted using the FACSTAR plus (Becton Dickinson). The cells were stained for human CD4 with a mouse antihuman CD4 PE antibody and the top 2-3% expressing cells were selected.
Immunizations were conducted as described in Example 1 using 1 X $10^7$ cells per mouse except that the primary injection was subcutaneous at the base of the neck. The mice received 6 injections 2-3 weeks apart. Serum was prepared and analyzed by ELISA as described in Example 1 except that the initial coating of the ELISA plate utilized 100 $\mu$l per well of recombinant soluble CD4 at 2 $\mu$g/ml of coating buffer. The titration curve for serum from XenoMouse™ after 6 injections is shown in Figure 5. Titers of human anti-CD4 reactivity were shown at concentrations representing greater than those of 1:1,000 dilution.

Example 4

Human Antibodies Against Human L-selectin

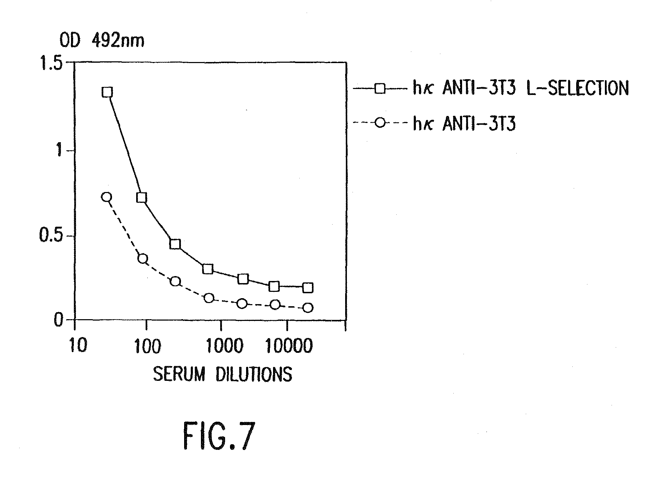

The antigen was prepared as a surface displayed protein in C51 cells, a high expressing clone derived by transfecting the mouse pre-B cell 300.19 with LAM-1 cDNA (LAM-1 is the gene encoding L-selectin) (Tedder, et al., J. Immunol (1990) 144:532) or with similarly transfected CHO cells. The transfected cells were sorted using fluorescent activated cell sorting using anti-Leu-8 antibody as label.
The C51 and the transfected CHO cells were grown in DME 4.5 g/l glucose with 10% FCS and 1 mg/ml G418 in 100 mm dishes. Negative control cells, 3T3-P317 (transfected with gag/pol/env genes of Moloney virus) were grown in the same medium without G418.
Primary immunization was done by injection subcutaneously at the base of the neck; subsequent injections were intraperitoneal. 70-100 million C51 or transfected CHO cells were used per injection for a total of five injections 2-3 weeks apart.
Sera were collected as described in Example 1 and analyzed by ELISA in a protocol similar to that set forth in Example 1.
For the ELISA, the transfected cells were plated into 96 well plates and cell monolayers grown for 1-2 days depending on cell number and used for ELISA when confluent. The cells were fixed by first washing with cold 1 x PBS and then fixing solution (5% glacial acetic acid, 95% ethanol) was added. The plates were incubated at -25˚C for 5 minutes and can be stored at this temperature if sealed with plate sealers.
The ELISA is begun by bringing the plates to room temperature, flicking to remove fixing solution and washing 5 times with DMEM medium containing 10% FCS at 200 $\mu$l per well.
The wells were treated with various serum dilutions or with positive or negative controls. Positive control wells contained murine IgGl monoclonal antibody to human L-selectin.
The wells were incubated for 45 minutes and monolayer integrity was checked under a microscope. The wells were then incubated with antihuman $\kappa$ chain antibody or antihuman $\mu$ chain antibody conjugates with HRP described in Example 1. The plates were then washed with 1% BSA/PBS and again with PBS and monolayer integrity was checked. The plates were developed, stopped, and read as described above. The results for serum from XenoMouse™ are shown in Figures 6 and 7; human antibodies both to L-selectin and control 3T3 cells were obtained. However, the serum titers are higher for the L-selectin-expressing cells as compared to parental 3T3 cells. These results show the XenoMause™ produces antibodies specific for L-selectin with human $\mu$ heavy chain regions and human $\kappa$ light chains.
The antisera obtained from the immunized XenoMouse™ were also tested for staining of human neutrophils which express L-selectin. Human neutrophils were prepared as follows:

peripheral blood was collected from normal volunteers with 100 units/ml heparin. About 3.5 ml blood was layered over an equal volume of One-step Polymorph Gradient (Accurate Chemical, Westbury, NY) and spun for 30 minutes at 450 x g at 20°C. The neutrophil fraction was removed and washed twice in DPBS/2% FBS.

The neutrophils were then stained with either;

(1) antiserum from XenoMouse™ immunized with C51 cells (expressing L-selectin);
(2) as a negative control, antiserum from a XenoMouse™ immunized with cells expressing human gp39.

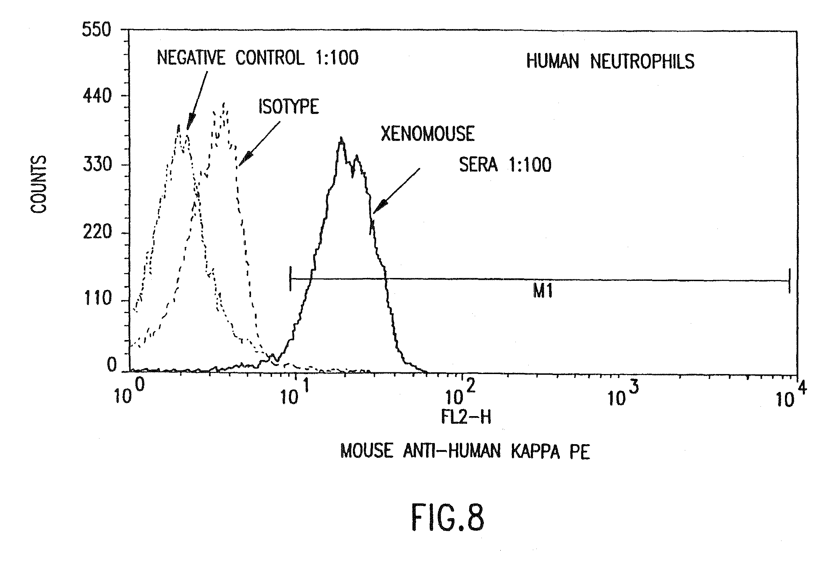

The stained, washed neutrophils were analyzed by FACS. The results for antiserum from XenoMouse™ are shown in Figure 8.

These results show the presence of antibodies in immunized XenoMouse™ serum which contain fully human light chains immunoreactive with L-selectin. The negative control antiserum from mice immunized with gp39 does not contain antibodies reactive against human neutrophils.

Example 5

Human Antibodies Against Human gp39

gp39 (the ligand for CD40) is expressed on activated human CD4 T cells. The sera of XenoMouse™ immunized with recombinant gp39 according to this example contained fully human antibodies immunospecific for gp39.

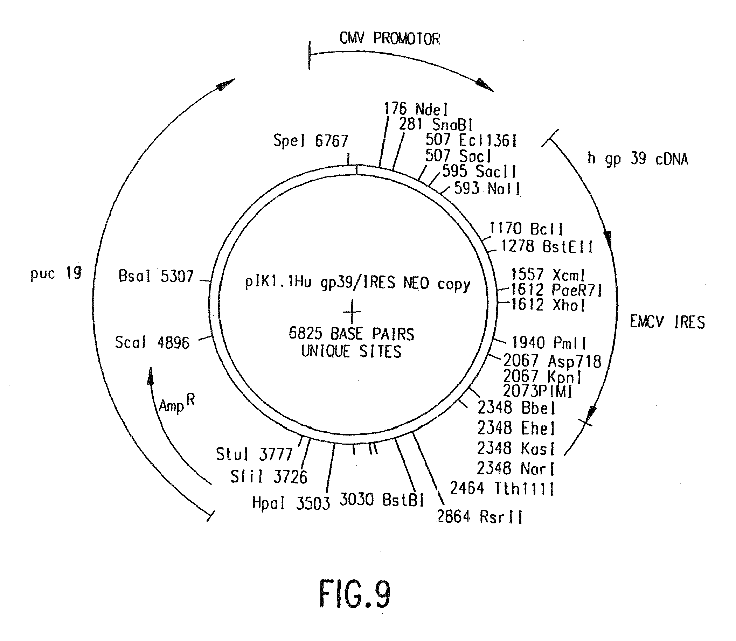

The antigen consisted of stable transfectants of 300.19 cells or of CHO cells expressing gp39 cDNA cloned into the mammalian expression vector P1K1.HUgp39/IRES NEO as shown in Figure 9. CHO cells were split 1:10 prior to transfection in DMEM 4.5 g/l glucose, 10% FBS, 2 mM glutamine, MEM, NEAA supplemented with additional glycine, hypoxanthine and thymidine. The cells were cotransfected with the gp39 vector at 9 $\mu$g/10 cm plate (6 x $10^5$ cells) and the DHFR expressing vector pSV2DHFRs (Subranani et al., Mol Cell Biol (1981) $\underline{9}$:854) at 1 $\mu$g/10 cm plate using calcium phosphate transfection. 24 hours later the cells were split 1:10 into the original medium containing G418 at 0.6 mg/ml. Cells producing gp39 were sorted by FACS using an anti-gp39 antibody.

Mice grouped as described in Example 1 were immunized with 300.19 cells expressing gp39 using primary immunization subcutaneously at the base of the neck and with secondary intraperitoneal injections every 2-3 weeks. Sera were harvested as described in Example 1 for the ELISA assay. The ELISA procedure was conducted substantially as set forth in Example 1; the microtiter plates were coated with CHO cells expressing gp39 grown in a 100 mm dish in DMEM, 4.5 g/1 glucose, 10% FCS, 4mM glutamine, and nonessential amino acid (NEAA) solution for MEM (100X). On the day preceding the ELISA assay, the cells were trypsinized and plated into well filtration plates at $10^5$ cells/200 $\mu$l well and incubated at 37°C overnight. The positive controls were mouse antihuman gp39; negative controls were antisera from mice immunized with an antigen other than gp39. 50 $\mu$l of sample were used for each assay. The remainder of the assay is as described in Example 1.

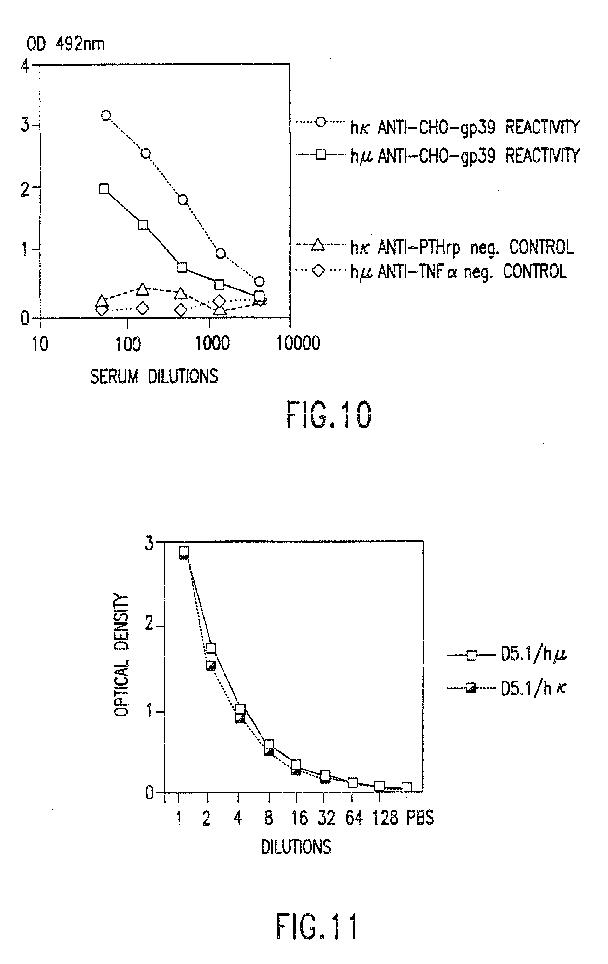

The dilution curves for the sera obtained after 4 injections from mice immunized with gp39 expressed on CHO cells are shown in Figure 10. As shown, the sera contained antihuman gp39 immunospecificity which is detectable with anti-human $\kappa$ and anti-human $\mu$ chain antibodies coupled to HRP.

Example 6

Preparation of Human Mabs Against Tetanus Toxin

The antibodies prepared in this example were secreted by hybridomas obtained by immortalizing B cells from xenomice immunized with tetanus toxin. The immunization protocol was similar to that set forth in Example 1 using 50 $\mu$g tetanus toxin emulsified in complete Freund's adjuvant for intraperitoneal primary immunization followed by subsequent intraperitoneal injections with antigen incorporated into incomplete Freund's adjuvant. The mice received a total of 4 injections 2-3 weeks apart.

After acceptable serum titers of antitetanus toxin C (anti-TTC) were obtained, a final immunization dose of antigen in PBS was give 4 days before the animals were sacrificed and the spleens were harvested for fusion.

The spleen cells were fused with myeloma cells P3X63-Ag8.653 as described by Galfre, G. and Milstein, C. Methods in Enzymology (1981) 73:3-46.

After fusion the cells were resuspended in DMEM, 15% FCS, containing HAT supplemented with glutamine, pen/strep for culture at 37°C and 10% $CO_2$. The cells were plated in microtiter plates and maintained in HAT-supplemented medium for two weeks before transfer to HAT-supplemented medium. Supernatants from wells containing hybridomas were collected for a primary screen using an ELISA.

The ELISA was conducted as described in Example 1 wherein the antigen coating consisted of 100 $\mu$l/well of

tetanus toxin C (TTC) protein at 2 µg/ml in coating buffer, followed by incubation at 4˚C overnight or at 37˚C for two hours. In the primary ELISA, HRP-conjugated mouse antihuman IgM was used as described in Example 1. Two hybridomas that secreted anti-TTC according to the ELISA assay, clone D5.1 and clone K4.1 were used for further analysis.

As shown in Figure 11, clone D5.1 secretes fully human anti-TTC which is detectable using HRP-conjugated antihuman µ chain antibody and HRP-conjugated antihuman κ chain antibody. This is confirmed in Figure 11.

The antibody secreted by D5.1 did not immunoreact in ELISAs using TNFα, IL-6, or IL-8 as immobilized antigen under conditions where positive controls (sera from xenomice immunized with TNFα, IL-6 and IL-8 respectively) showed positive ELISA results.

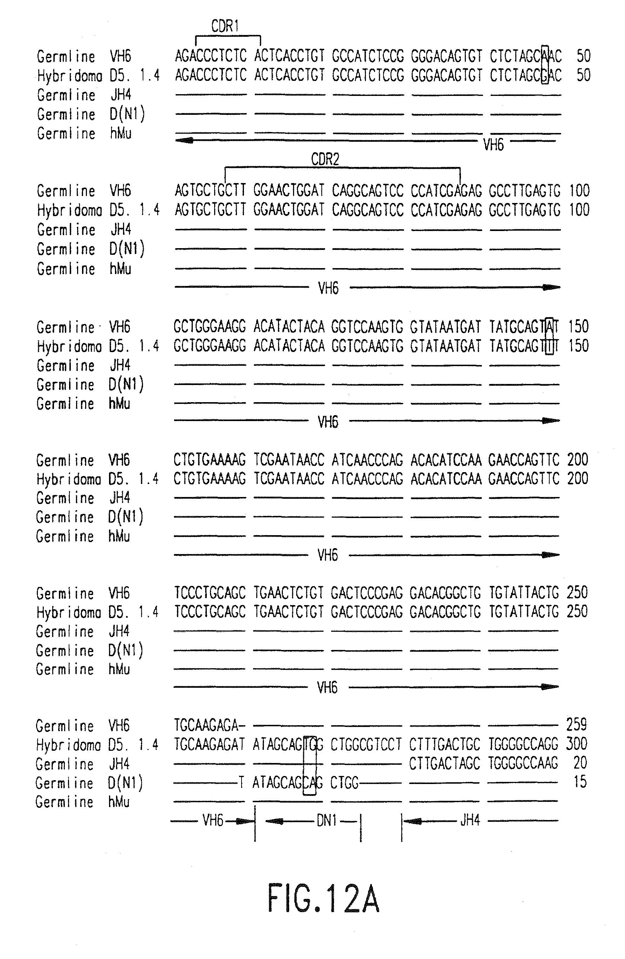

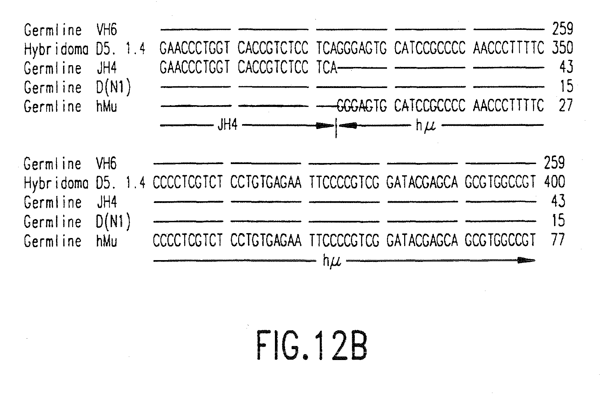

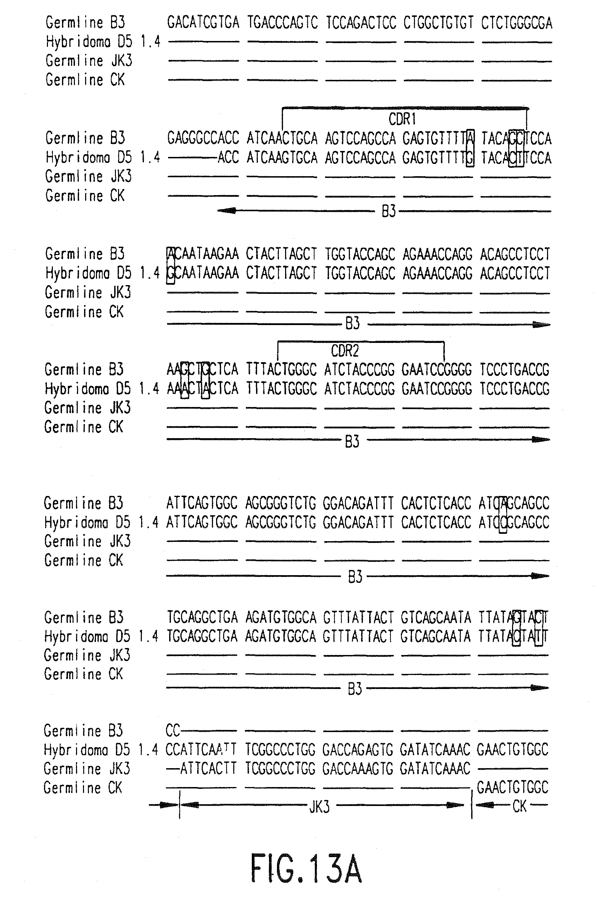

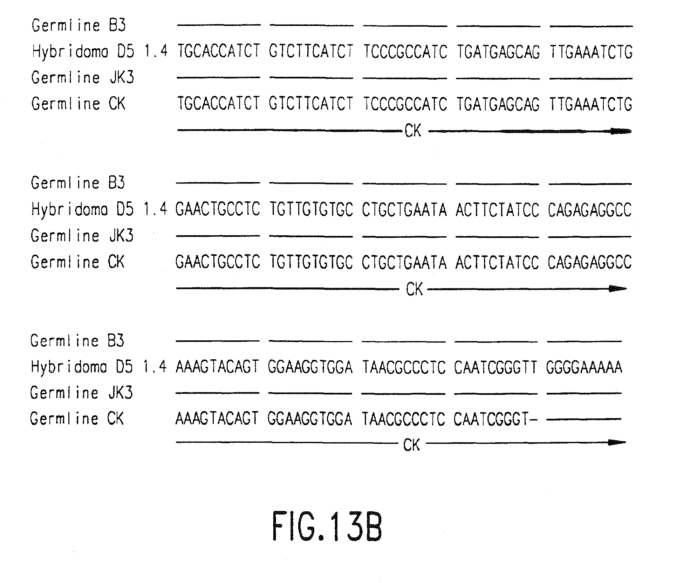

The complete nucleotide sequence of the cDNAs encoding the heavy and light chains of the monoclonal were determined as shown in Figures 12 and 13. polyA mRNA was isolated from about $10^6$ hybridoma cells and used to generate cDNA using random hexamers as primers. Portions of the product were amplified by PCR using the appropriate primers.

The cell line was known to provide human κ light chains; for PCR amplification of light chain encoding cDNA, the primers used were HKP1 (5'-CTCTGTGACACTCTCCTGGGAGTT-3') for priming from the constant region terminus and two oligos, used in equal amounts to prime from the variable segments; B3 (5'-GAAACGACACTCACGCAGTCTCCAGC-3').

For amplification of the heavy chain of the antibody derived form D5.1 (which contains the human µ constant region), MG-24VI was used to prime from the variable and µP1 (5'-TTTTCTTTGTTGCCGTTGGGGTGC-3') was used to prime from the constant region terminus.

Referring to Figure 12 which sets forth the sequence for the heavy chain of the antibody secreted by clone D5.1, this shows the heavy chain is comprised of the human variable fragment VH6, the human diversity region DN1 and the human joining segment JH4 linked to the human µ constant region. There were two base-pair mutations from the germline sequence in the variable region, both in the CDRs. Two additional mutations were in the D segment and six nongermline nucleotide additions were present at the $D_b$-$J_b$ junction.

Finally, referring to Figure 13 which presents the light chain of the antibody secreted by D5.1, the human x variable region B3 and human κ joining region JK3 are shown. There are nine base-pair differences from the germline sequences, three falling with CDR1.

Example 7

Human Antibodies Against PTHrp

Groups of XenoMouse™-2 were immunized intraperitoneally with either PTHrp (1-34) conjugated with BTG, as described by Ratcliffe et al., J. Immunol. Methods 127:109 (1990), or with PTHrp (1-34) synthesized as a 4 branched-MAP (multiple antigenic peptide system). The antigens were emulsified in CFA (complete Freunds adjuvant) and injected i.p. at a dose of 25 µg per animal at 2 week intervals, and bled after two injections. The sera obtained from this bleed were analyzed by ELISA as described supra.

Serum titers for hγ, hµ, and hκ after one immunization of the XenoMouse™ are shown in Table 2. When immunized with PTHrp, the XenoMouse™ showed low serum titers in 5 of 7 mice on the first bleed, but when PTHrp-MAP is used, 7 of 7 mice show high serum titers on the first bleed.

TABLE 1. AntiPTHrp serum titer responses of Xenomouse-2.

| First bleed after 2 immunizations with either PTHrp-BTG conjugate | | | |
|---|---|---|---|
| **XM2 PTHrp-BTG Conjugate** | **Human Responses** | | |
| | titer (via hγ) | titer (via hµ) | titer (via hκ) |
| 1 | <30 | 850 | 100 |
| 2 | <30 | 3,000 | 50 |
| 3 | <30 | 7,000 | 1,000 |
| 4 | <30 | 800 | 200 |
| 5 | <30 | 400 | 90 |
| 6 | <30 | 500 | 50 |
| 7 | <30 | 300 | 50 |

(continued)

| XM2 PTHrp-MaP | titer (via hγ) | titer (via hμ) | titer (via hκ) |
|---|---|---|---|
| 1 | <30 | 1,000 | 50 |
| 2 | <30 | 2,500 | 300 |
| 3 | <30 | 1,200 | 150 |
| 4 | 150 | 1,000 | 270 |
| 5 | 100 | 2,500 | 300 |
| 6 | <30 | 1,000 | 150 |
| 7 | <30 | 4,000 | 800 |

Example 8

Human Antibodies Against Human IL-8

Immunization and serum preparation were as described in Example 1 except that human recombinant IL-8 was used as an immunogen.

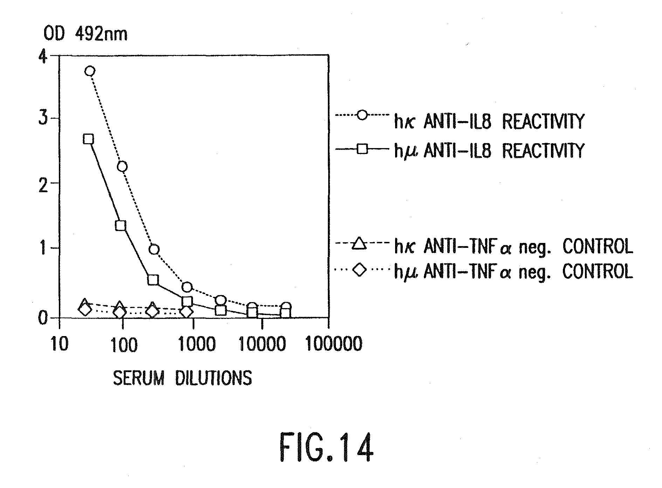

ELISA assays were performed with respect to the recovered serum, also exactly as described in Example 1, except that the ELISA plates were initially coated using 100 μl/well of recombinant human IL-8 at 0.5mg/ml in the coating buffer. The results obtained for various serum dilutions from XenoMouse™ after 6 injections are shown in Figure 14. Human anti-IL-8 binding was again shown at serum dilutions having concentrations higher than that represented by a 1:1,000 dilution.

Example 9

preparation of High Affinity Human Monoclonal Antibodies Against Human IL-8

Groups of 4 to 6 XenoMouse™ aged between 8 to 10 weeks old were used for immunization and for hybridoma generation. XenoMouse™ were immunized intraperitoneally with 25 μg of human recombinant-IL-8 (Biosource International, CA, USA) emulsified in complete Freund's adjuvant (CFA, Sigma) for the primary immunization. All subsequent injections were done with the antigen incorporated into incomplete Freund's adjuvant (IFA, Sigma). For animals used as spleen donors for hybridoma generation a final dose of antigen in phosphate buffer saline (PBS) was given 4 days before the fusion. Serum titers of immunized XenoMouse™ were first analyzed after a secondary dose of antigens, and from there after, following every antigen dose. Test bleeds were performed 6 to 7 days after the injections, by bleeding from the retrobulbar plexus. Blood was allowed to clot at room temperature for about 2 hours and then incubated at 4⎕C for at least 2 hours before separating and collecting the sera.

Generation of hybridomas

Spleen cells obtained from XenoMouse™ previously immunized with antigen, were fused with the non secretory NSO myeloma cells transfected with *bcl-2* (NSO-bcl2) as described in Galfre G, et al., Methods in Enzymology 73, 3-46, (1981). Briefly, the fusion was performed by mixing washed spleen cells and myeloma cells at a ratio of 5:1 and gently pelleting them by centrifugation at 800Xg. After complete removal of the supernatant the cells were treated with 1 ml of 50% PEG/DMSO (polyethylene glycol MW 1500, 10% DMSO, Sigma) which was added over 1 min., the mixture was further incubated for one minute, and gradually diluted with 2 ml of DMEM over 2 minutes and diluted further with 8 ml of DMEM over 3 minutes. The process was performed at 37⎕C with continued gentle stirring. After fusion the cells were resuspended in DMEM, 15% FCS, containing HAT, and supplemented with L glutamine, pen/strep, for culture at 37 QC and 10% CO2 in air. Cells were plated in flat bottomed 96 well microtiter trays. Cultures were maintained in HAT supplemented media for 2 weeks before transfer to HT supplemented media. Cultures were regularly examined for hybrid cell growth, and supernatants from those wells containing hybridomas were collected for a primary screen analysis for the presence of human μ, human gamma 2, and human kappa chains in an antigen specific ELISA as described above. Positive cultures were transferred to 48 well plates and when reaching confluence transferred to 24 well plates. Supernatants were tested in an antigen specific ELISA for the presence of human μ, human gamma 2, and human kappa chains.

As shown in Table 3 several hybridomas secreting fully human monoclonal antibodies with specificity for human IL-8 have been generated from representative fusions. In all of these human monoclonal antibodies the human gamma-2 heavy chain is associated with the human kappa light chain.

TABLE 3: ELISA determination of heavy and light chain composition of anti-IL-8 human monoclonal antibodies generated in XenoMouse™

| Sample ID | Ig class | reactivity to hIL8 | | | | Total hIgG |
|---|---|---|---|---|---|---|
| | | titers | $\underline{H}_\kappa$ OD (1:1) | m$\lambda$ OD (1:1) | h$\gamma$ OD (1:1) | (ng/ml) |
| Bkgd | | | 0.08 | 0.04 | 0.12 | |
| I8D1.1 | hIgG2 | 500 | 4.12 | 0.04 | 4.09 | 1,159 |
| I8K2.1 | hIgG2 | 200 | 4.18 | 0.18 | 4.11 | 2.000 |
| I8K2.2 | hIgG2 | 1,000 | 4.00 | 0.04 | 4.00 | 4,583 |
| I8K4.2 | hIgG2 | 200 | 3.98 | 0.04 | 3.49 | 450 |
| I8K4.3 | hIgG2 | 200 | 3.80 | 0.05 | 4.09 | 1,715 |
| I8K4.5 | hIgG2 | 1,000 | 4.00 | 0.06 | 4.00 | 1,468 |

Evaluation of kinetic constants of XenoMouse™ hybridomas

In order to determine the kinetic parameters of these antibodies, specifically their on and off rates and their dissociation constants (KD), they were analyzed on the BIAcore instrument (Pharmacia). The BIAcore instrument uses plasmon resonance to measure the binding of an antibody to an antigen-coated gold chip.

BIAcore reagents and instrumentation:

The BIAcore instrument, CM5 sensor chips, surfactant P20, and the amine coupling kit containing N-hydroxysuccinimide (NHS), N-ethyl-N[1]-(3-diethylaminopropyl)-carbodimide (EDC), and ethanolamine were purchased from Pharmaicia Biosensor. Immobilization of human recombinant IL-8 onto the sensor surface was carried out at low levels of antigen density immobilized on the surface and was performed according to the general procedures outlined by the manufacturers. Briefly, after washing and equilibrating the instrument with HEPES buffer (HBS; 10 mM HEPES, 150 mM NaCl, 0.05% surfactant P20, pH 7.4) the surface was activated and IL-8 immobilized for the subsequent binding and kinetic studies. The sensor surface was activated with 5 $\mu$l of a mixture of equal volumes of NHS (0.1 M) and EDC (0.1 M) injected at 10 $\mu$l/min across the surface for activation, then 5 $\mu$l of the ligand (human recombinant IL-8) at 12 $\mu$g/ml in 5 mM maleate buffer, pH 6.0 was injected across the activated surface, and finally non-conjugated active sites were blocked with an injection of 35 $\mu$l of 1 M ethanolamine. The surface was washed to remove non-covalently bound ligand by injection of 5 $\mu$l 0.1 M HCl. All the immobilization procedure was carried out with a continuous flow of HBS of 10 $\mu$l/min. About 100 resonance units (RU) of ligand (82 and 139 RU, in separate experiments) were immobilized on the sensorship, (according to the manufacturers 1,000 RU corresponds to about 1 ng/mm$^2$ of immobilized protein).
These ligand coated surfaces were used to analyze hybridoma supernatants for their specific binding to ligand and for kinetic studies. The best regenerating condition for the analyte dissociation from the ligand in these sensorships was an injection of 10 $\mu$l 100 mM HCl with no significant losses of binding observed after many cycles of binding and regeneration.
Determination of the dissociation, and association rates and the apparent affinity constants of fully human monoclonal antibodies specific for IL-8.
The determination of kinetic measurements using the BIAcore in which one of the reactants is immobilized on the sensor surface was done following procedures suggested by the manufacturers and described in Karlsson et al. "Kinetic analysis of monoclonal antibody-antigen interaction with a new biosensor based analytical system." J. Immunol. Methods (19910 145, 229. Briefly the single site interaction between two molecules A and B is described by the following equation.

$$d[AB]/dt=ka[A][B]-kd[AB]$$

In which B is immobilized on the surface and A is injected at a constant concentration C. The response is a measure of the concentration of the complex [AB] and all concentration terms can be expressed as Response Units (RU) of the BIAcore:

$$dR/dt-kaC(Rmax-R) - kdR$$

where dR/dt is the rate of change of the signal, C is the concentration of the analyte, Rmax is the maximum analyte binding capacity in RU and R is the signal in RU at time t. In this analysis the values of ka and kd are independent of the concentration of immobilized ligand on the surface of the sensor. The dissociation rates (kd) and association rates (ka) were determined using the software provided by the manufacturers, BIA evaluation 2.1. The dissociation rate constant was measured during the dissociation phase that extended for 10 minutes at a constant buffer flow rate of 45 ul/min, after the completion of the injection of the hybridoma supernatants onto the surface containing immobilized IL-8. The association phase extended over 1.25 minutes at a flow rate of 45 ul/min and the data was fitted into the model using the previously determined kd values. At least two surfaces with different levels of immobilized ligand were used in which different concentrations of anti IL-8 hybridoma supernatants were tested for binding and analyzed for kinetic data. The kinetic constants determined on these two surfaces are presented in Table 4. The affinities were determined to be very, ranging from $7 \times 10^{-11}$ to $2 \times 10^{-9}$ M. This compares vary favorably with the affinities of murine monoclonal antibodies derived from normal mice.

TABLE 4: Kinetic constants of fully human monoclonal antibodies (IgG2, kappa) derived from XenoMouse™ II-a with specificity to human IL-8, determined by BIAcore.

| Hybridoma | association rate ka $(M^{-1}_s{}^{-1})$ | dissociation rate kd $(_s{}^{-1})$ | Dissociation Constant KD (M) =kd/ka | BIAcore surface h-IL-8 [RU] |
|---|---|---|---|---|
| I8D1-1 | 3.36 x 106 <br> 2.80 x 106 | 2.58 x 10-4 <br> 1.73 x 10-4 | 7.70 x 10-11 <br> 6.20 x 10-11 | 81 <br> 134 |
| I8K2-1 | 4.38 x 105 <br> 3.83 x 105 | 6.73 x 10-4 <br> 6.85 x 10-4 | 1.54 x 10-9 <br> 1.79 x 10-9 | 81 <br> 134 |
| I8K2-2 | 5.24 x 105 <br> 4.35 x 105 | 2.26 x 10-4 <br> 2.30 x 10-4 | 4.30 x 10-10 <br> 5.30 x 10-10 | 81 <br> 134 |
| I8K4-2 | 5.76 x 106 <br> 1.95 x 106 | 8.17 x 10-4 <br> 3.84 x 10-4 | 1.42 x 10-10 <br> 1.96 x 10-10 | 81 <br> 134 |
| I8K4-3 | 2.66 x 106 <br> 1.46 x 106 | 7.53 x 10-4 <br> 5.72 x 10-4 | 2.83 x 10-10 <br> 3.90 x 10-10 | 81 <br> 134 |
| I8K4-5 | 4.00 x 105 <br> 1.70 x 105 | 9.04 x 10-4 <br> 4.55 x 10-4 | 2.26 x 10-9 <br> 2.68 x 10-9 | 81 <br> 134 |

Methods for isolation of human neutrophils and assays for antibody activity

The primary in vivo function of IL-8 is to attract and activate neutrophils. Neutrophils express on their surface two distinct receptors for IL-8, designated the A receptor and the B receptor. In order to determine whether the fully human antibodies could neutralize the activity of IL-8, two different in vitro assays were performed with human neutrophils. In one assay, the ability of the antibodies to block binding or radiolabelled IL-8 to neutrophil IL-8 receptors was tested. In a second assay, the antibodies were tested for their ability to block an IL-8-induced neutrophil response, namely the upregulation of the integrin Mac-1 on the neutrophil surface. Mac-1 is composed of two polypeptide chains, CD11b and CD18. Typically, anti-CD11b antibodies are used for its detection.

Isolation of neutrophils:

Human neutrophils are isolated from either freshly drawn blood or buffy coat. Human blood is collected by venipuncture into sterile tubes containing EDTA. Buffy coats are obtained from Stanford Blood Bank. They are prepared by centrifuging anticoagulated blood (up to 400 ml) in plastic bags at 2600 xg for 10 min at 20˚C with the brake off. The plasma supernatant is aspirated out of the bag and the buffy coat, i.e., the upper cell layer (40-50 ml/bag) is collected. One unit of buffy coat (40-50 ml) is diluted to final volume of 120 ml with $Ca^{2+}$, $Mg^{2+}$-free PBS. 30 milliliters of blood or diluted buffy coat are transferred into 50-ml centrifuge tubes on top of a 20-ml layer of Ficoll-Paque Plus (Pharmacia Biotech). The tubes are centrifuged at 500 xg for 20 min at 20˚C with brake off. The supernatant, the mononuclear cells at the interface, and the layer above the pellet are carefully withdrawn. To completely remove the mononuclear cells, the cell pellet containing neutrophils and erythrocytes is resuspended with 5 ml of PBS and transferred into clean 50-ml tubes. The cells are washed in $Ca^{2+}$, $Mg^{2+}$-free PBS (300 xg for 5 min at 4˚C). The erythrocytes are then lysed with ammonium chloride. The cells are resuspended in 40 ml of an ice-cold solution containing 155 mM $NH_4Cl$ and 10 nM EDTA, pH 7.2-7.4. The tubes are kept on ice for 10 min with occasional mixing and then centrifuged at 300 xg for 5 min at 4˚C. The pellet is resuspended in PBS and washed once (300 xg for 5 min at 4˚C). If erythrocyte lysis appears incomplete, the treatment with ammonium chloride is repeated. The neutrophils are again washed and finally suspended either in assay medium (RPMI-1640 supplemented with 10% fetal calf serum, 2 mM L-glutamine, $5x10^{-5}$ 2-mercaptoethanol, 1X nonessential amino acids, 1 mM sodium pyruvate and 10 mM Hepes) at a density of $3x10^7$ cells/ml or in a binding buffer (PBS containing 0.1% bovine serum albumin and 0.02% $NaN_3$), at a density of $6x10^6$ cells/ml.

IL-8 receptor binding assay:

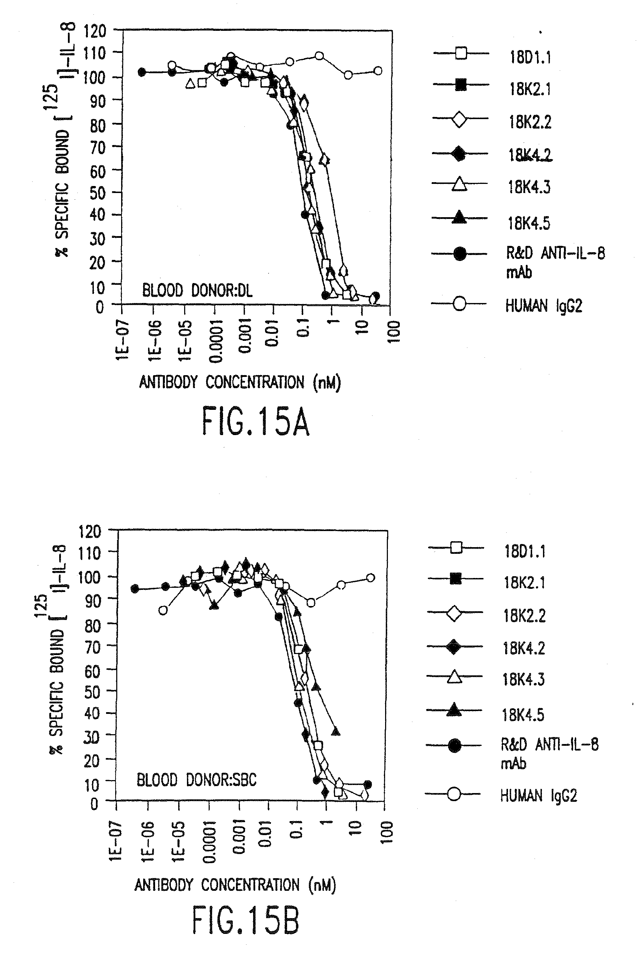

Multiscreen filter plates (96-well, Millipore, MADV N6550) were pretreated with a PBS binding buffer containing 0.1% bovine serum albumin and 0.02% $NaN_3$ at 25˚C for 2 hours. A final volume of 150 $\mu$l, containing $4x10^5$ neutrophils, 0.23 nM $[^{125}I]$-human-IL-8 (Amersham, IM-249) and varying concentrations of antibodies made up in PBS binding buffer, was added to each well, and plates were incubated for 90 min at 4˚C. Cells were washed 5 times with 200 $\mu$l of ice-cold PBS, which was removed by aspiration. The filters were air-dried, 3.5 ml of scintillation fluid was added (Beckman Ready Safe) and filters were counted on a Beckman LS6000IC counter. The data obtained is presented as % specific bound $[I^{125}]$-IL-8, which is calculated as the cpm in the presence of antibody divided by the cpm in the presence of PBS binding buffer only and multiplied by 100 (Figure 15). All six of the human anti-IL-8 monoclonals tested blocked IL-8 binding to human neutrophils.

Neutrophil CD11b (Mac-1) expression assay:

Human IL-8 at a final concentration of 10 nM was preincubated with varying concentrations of monoclonal antibodies at 4˚C for 30 minutes and at 37˚C for an additional 30 min. Neutrophils ($4x10^5$/well) were exposed to IL-8 in the presence or absence of antibodies at 4˚C for 90 min, and incubated with PE-conjugated mouse-anti-human-CD11b (Becton Dickinson) for 45 min at 4˚C. The cells were washed with ice-cold PBS containing 2% fetal calf serum. Fluorescence was measured on a Becton Dickinson FACscan cell analyzer. A mouse monoclonal antibody against human CD11b obtained from R&D System, Inc. was used as a positive control while the purified myeloma human IgG2 (Calbiochem) was used as a negative control in the experiments. The expression levels of CD11b on neutrophils were measured and expressed as the mean fluorescence channel. The mean fluorescence channel derived form the negative control antibody was subtracted from those of experimental samples.

```
                     mean fluorescence in     mean fluorescence in
                     presence of IL-8      -  the presence of
                     only                     antibodies
    % inhibition = --------------------------------------------- x 100
                     mean fluorescence in     mean fluorescence in
                     the presence of IL-8  -  the presence of
                     only                     human IgG2
```

As shown in Table 5, five of the six antibodies blocked upregulation of CD11b to some degree, with three of the five giving complete blocking.

TABLE 5: Inhibition of CD11b expression on human neutrophils by monoclonal antibodies against IL-8.

| Antibody | Concentration (nM) | Inhibition of CD11b expression (%) |
|---|---|---|
| R&D anti-IL8 | 333 | 100 |

(continued)

| Antibody | Concentration (nM) | Inhibition of CD11b expression (%) |
|---|---|---|
| I8K1.1 | 6 | 100 |
| I8K2.1 | 10 | 60 |
| I8K2.2 | 32 | 100 |
| I8K4.2 | 3 | 10 |
| I8K4.3 | 8 | 100 |
| I8K4.5 | 5 | 0 |
| Human IgG2 | 33 | 0 |

Background of CD11b expression is 670 (mean fluorescence) while CD11b expression in the presence of 10 nM of human IL-8 is 771.

Sequence analysis of Immunoglobulin transcripts derived from anti-hIL-8 hybridomas.

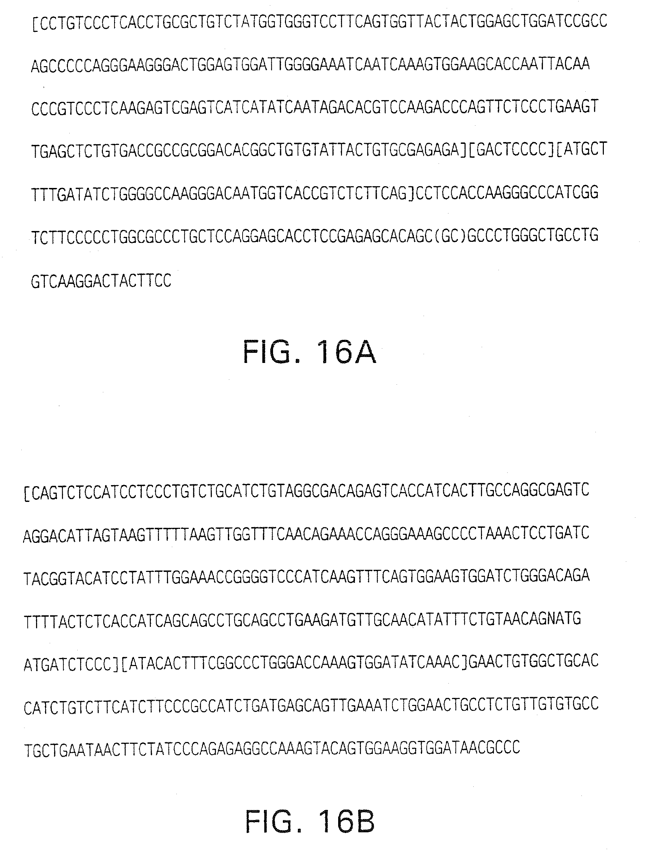

All sequences were derived by direct sequencing of PCR fragments generated form RT-PCR reactions of RNA prepared from hybridomas D1.1, K2.2, K4.2 and K4.3, using human $V_H$ and human $V_k$ family specific primers (Marks et. al. 1991; Euro J. Immunol 21;985-991) and a primer specific for either the human gamma 2 constant region (MG-40d; 5'GCTGAGGGAGTAGAGTCCTGAGGACTGT-3') or human kappa constant region (HKP2; Green et al 1994; Nature Genetics 7: 13-21)). In Figure 16A-H, both strands of the four clones were sequenced and analyzed to generate the complete sequence. All sequences were analyzed by alignments to the "V BASE sequence directory", Tomlinson et al., MRC Centre for Protein Engineering, Cambridge, UK. The variable and joining regions are indicated by brackets []. Nucleotides containing an "N" indicate uncertainty in the generated sequence.

Based on sequence alignments with sequences found in the V-base database the heavy chain transcript from hybridoma D1.1 has a human $V_H$4-21(DP-63) variable region (7 point mutations were observed compared to the germline sequence), a human 21-10rc D segment, a human $J_H$3 joining region and a human gamma 2 constant region. See Figure 16A.

The kappa light chain transcript from hybridoma D1.1 is comprised of a human kappa variable region with homology to $V_\kappa$ 08/018 (DPK1) (16 point mutations were observed when compared to the germline sequence) a human $J_\kappa$3 joining region, and a human kappa constant region. See Figure 16B.

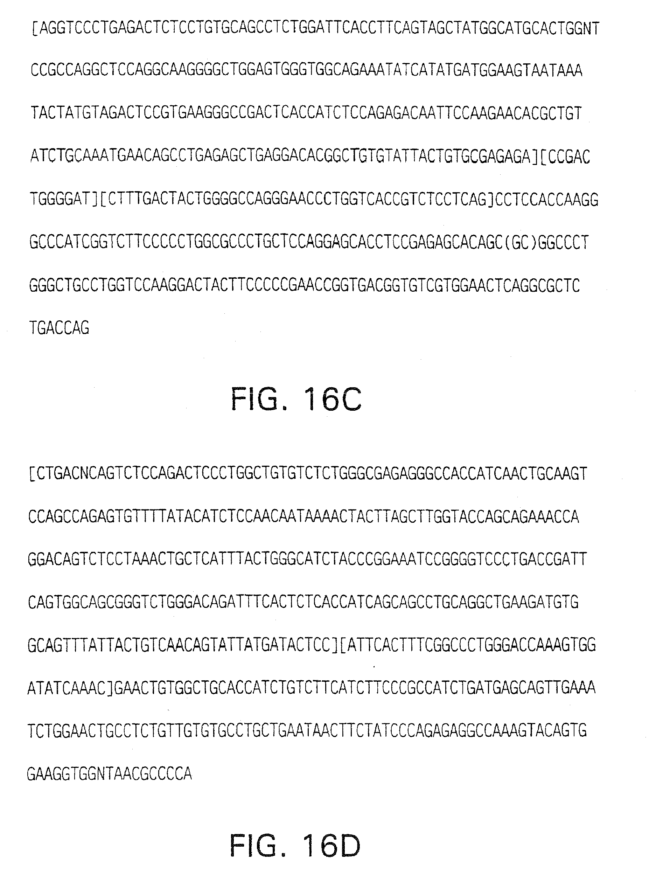

Based on sequence alignments with sequences found in the V-base database the heavy chain transcript from hybridoma K2.2 has a human $V_H$3-30 variable region (3 point mutations were observed compared to the germline sequence), a human IR3rc D segment, a human $J_H$4 joining region and a human gamma 2 constant region. See Figure 16C.

The kappa light chain transcript from hybridoma K2.2 is comprised of a human kappa variable region with homology to $V_k$IV (B3; DPK24) (9 point mutations were observed when compared to the germline sequence), a human $J_K$3 joining region, and a human kappa constant region. See Figure 16D.

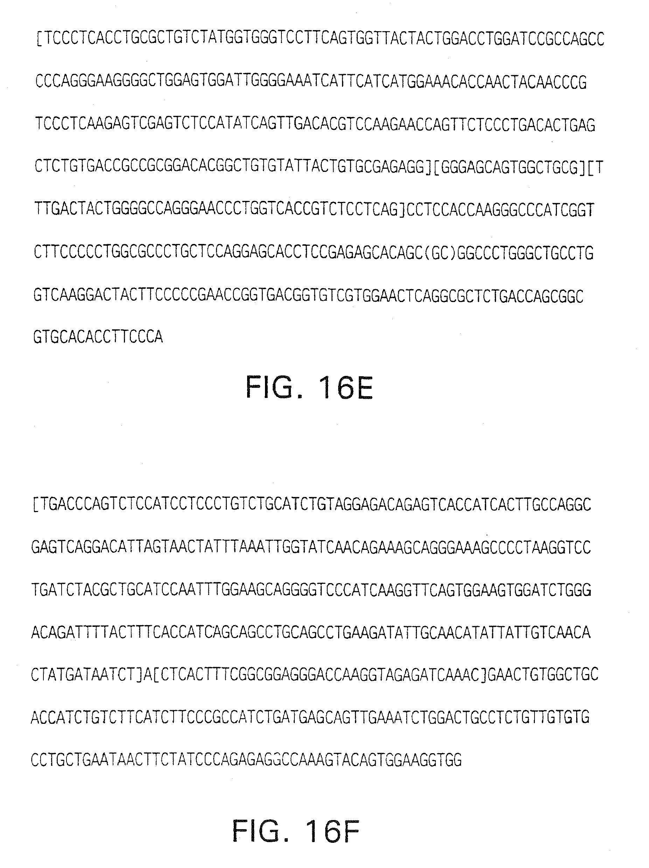

Based on sequence alignments with sequences found in the V-base database the heavy chain transcript from hybridoma K4.2 has a human $V_H$4-34 variable region (8 point mutations were observed compared to the germline sequence), a human K1 D segment, a human $J_H$4 joining region and a human gamma 2 constant region. See Figure 16E.

The kappa light chain transcript from hybridoma K4.2 is comprised of a human kappa variable region with homology to $V_\kappa$ 08/018 (DPK1) (6 point mutations were observed when compared to the germline sequence), a human $J_\kappa$4 joining region, and a human kappa constant region. See Figure 16F.

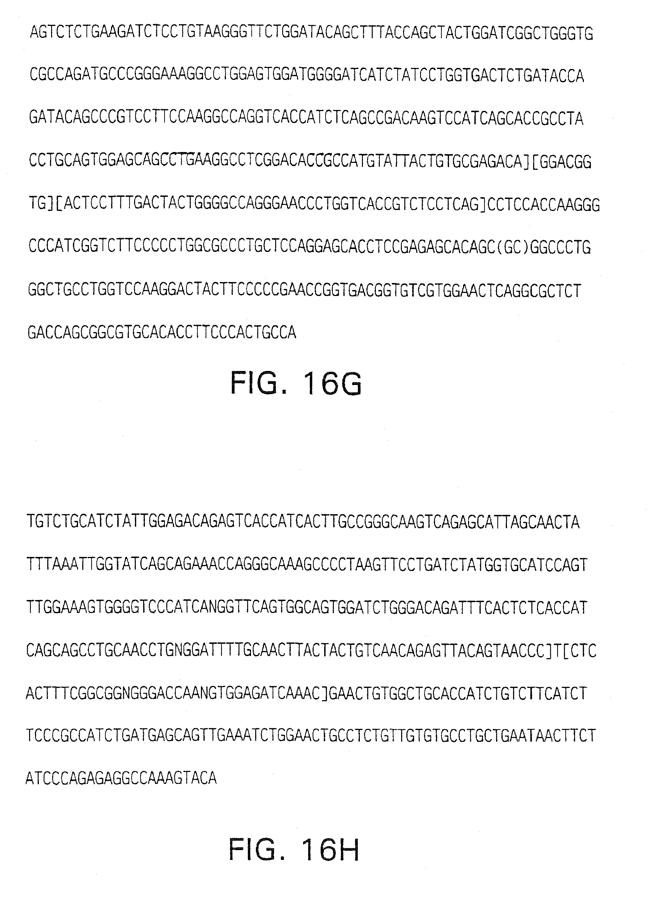

Based on sequence alignments with sequences found in the V-base database the heavy chain transcript from hybridoma K4.3 has a human $V_H$5-51 (DP-73) variable region, a human M5-a/M5-b D segment, a human $J_H$4 joining region and a human gamma 2 constant region. See Figure 16G.

The kappa light chain transcript from hybridoma K4.3 is comprised of a human kappa variable region with homology to $V_\kappa$ 02/012 (DPK9) (9 point mutations were observed when compared to the germline sequence), a human $J_\kappa$4 joining region, and a human kappa constant region. See Figure 16H.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

Biological Deposits

yH1C contained in *S. cerivisiae* was deposited with the American Type Culture Collection ("ATCC"), 12301 Parklawn Drive, Rockville MD 20852, USA, on April 26, 1996, and given ATCC accession no. 74367. The deposit of this YAC is for exemplary purposes only, and should not be taken as an admission by the Applicant that such deposit is necessary for enablement of the claimed subject matter.

In respect of all designated States in which such action is possible and to the extent that it is legally permissible under the law of the designated State, it is requested that a sample of the deposited micro-organism be made available only by the issue thereof to an independent expert, in accordance with the relevant patent legislation, e.g., EPC rule 28 (4), United Kingdom Patent Rules 1982 rule 17(3), Australian Regulation 3.25(3) and generally similar provisions *mutatis mutandis* for any other designated State.

International Application No: PCT/

---

## MICROORGANISMS

Optional Sheet in connection with the microorganism referred to on page 41, lines 1-20 of the description '

**A. IDENTIFICATION OF DEPOSIT** '

Further deposits are identified on an additional sheet '

Name of depositary institution '

**American Type Culture Collection**

Address of depositary institution (including postal code and country)

**12301 Parklawn Drive**
**Rockville, MD  20852**
**US**

Date of deposit ' <u>April 26, 1996</u>  Accession Number  _____

**B. ADDITIONAL INDICATIONS** ' (leave blank if not applicable). This information is continued on a separate attached sheet

**C. DESIGNATED STATES FOR WHICH INDICATIONS ARE MADE** ' (if the indications are not all designated States)

**D. SEPARATE FURNISHING OF INDICATIONS** ' (leave blank if not applicable)

The indications listed below will be submitted to the International Bureau later ' (Specify the general nature of the indications e.g , "Accession Number of Deposit")

E. ☑ This sheet was received with the International application when filed (to be checked by the receiving Office)

_H. Anton Smith, Paralegal_
(Authorized Officer)

☐ The date of receipt (from the applicant) by the International Bureau "

was _____
(Authorized Officer)

Form PCT/RO/134 (January 1981)

---

1. A method to produce a human immunoglobulin or an analog thereof, specific for a desired antigen, which method comprises:

administering said antigen or an immunogenic portion thereof to a nonhuman animal under conditions to stimulate an immune response, whereby said animal produces B cells that secrete immunoglobulin specific for said antigen; wherein said nonhuman animal is characterized by being substantially incapable of producing endogenous heavy and light immunoglobulin chains, but capable of producing human immunoglobulin; and recovering said immunoglobulin or analog.

2. The method of item 1 wherein said recovering step comprises recovering polyclonal immunoglobulin or analog from said animal.

3. The method of item 1 wherein said recovering step comprises immortalizing B cells from said animal immunized with said antigen, screening the resulting immortalized cells for the secretion of said immunoglobulin specific for said antigen, and

a) recovering immunoglobulin secreted by said immortalized B cells, or
b) recovering the genes encoding at least the immunoglobulin from the immortalized B cells, and optionally modifying said genes;

expressing said genes or modified forms thereof to produce immunoglobulin or analog; and
recovering said immunoglobulin or analog.

4. The method of item 1 wherein said recovering step comprises:

recovering genes encoding the immunoglobulins from the primary B cells of the animal;
generating a library of said genes expressing the immunoglobulins;
screening the library for an immunoglobulin with the desired affinity for the antigen;
recovering the genes encoding the immunoglobulin;
expressing said recovered genes to produce an immunoglobulin or analog recovering said immunoglobulin or analog.

5. A recombinant DNA molecule comprising a nucleotide sequence encoding the immunoglobulin or analog produced by the method of item 1.

6. The DNA molecule of item 5 wherein said encoding-nucleotide sequence is operably linked to control sequences capable of effecting its expression.

7. A cell or cell line modified to contain the DNA molecule of item 6.

8. A method to produce a fully human immunoglobulin or an analog thereof which method comprises culturing the cells of item 7 under conditions whereby said encoding nucleotide sequence is expressed to produce said immunoglobulin or analog; and recovering said immunoglobulin or analog.

9. A DNA molecule comprising a nucleotide sequence corresponding to the gene or modified gene prepared by the method of item 3.

10. The DNA molecule of item 9 wherein said encoding nucleotide sequence is operably linked to control sequences capable of effecting its expression.

11. A cell or cell line modified to contain the DNA molecule of item 9.

12. A method to produce a fully human immunoglobulin or an analog thereof which method comprises culturing the cells of item 11 under conditions whereby said encoding nucleotide sequence is expressed to produce said immunoglobulin or analog; and recovering said immunoglobulin or analog.

13. A DNA molecule which comprises a nucleotide sequence encoding a human immunoglobulin with desired affinity prepared according to the method of item 4.

14. The DNA molecule of item 13 wherein said encoding nucleotide sequence is operably linked to control sequences capable of effecting its expression.

15. A cell or cell line modified to contain the DNA molecule of item 13.

16. A method to produce a fully human immunoglobulin or an analog thereof which method comprises culturing the cells of item 15 under conditions whereby said encoding nucleotide sequence is expressed to produce said immunoglobulin or analog; and recovering said immunoglobulin or analog.

17. An immortalized B cell which secretes a fully human immunoglobulin to a desired antigen prepared according to item 3.

18. A method to produce an immunoglobulin or analog which comprises culturing the cells of item 17 and recovering said immunoglobulin or analog.

19. A fully human immunoglobulin or analog produced by the method of item 1.

20. The immunoglobulin or analog of item 19 wherein the desired antigen is selected from the group consisting of the leukocyte markers, CD2, CD3, CD4, CDS, CD6, CD7, CD8, CD11a,b,c, CD13, CD14, CD18, CD19, CD20, CD22, CD23, CD27 and its ligand, CD28 and its ligands B7.1, B7.2, B7.3, CD29 and its ligand, CD30 and its ligand, CD40 and its ligand gp39, CD44, CD45 and isoforms, CDw52 (Campath antigen), CD56, CD58, CD69, CD72, CTLA-4, LFA-1 and TCR;

the histocompatibility antigens, MHC class I or II, the Lewis Y antigens, SLex, SLey, SLea, and SLeb;

the integrins, VLA-1, VLA-2, VLA-3, VLA-4, VLA-5, VLA-6, $\alpha V\beta 3$, and LFA-1, Mac-1, and pl50,95, $\alpha_v\beta_1$, gpIIbIIIa, $\alpha_R\beta_3$, $\alpha_6\beta_4$, $\alpha_v\beta_5$, $\alpha_v\beta_6$, and $\alpha_v\beta_7$;

the selectins, L-selectin, P-selectin, and E-selectin and their counterreceptors VCAM-1, ICAM-1, ICAM-2, and LFA-3;

the interleukins, IL-1, IL-2, IL-3, IL-4, IL-5, IL-6, IL-7, IL-8, IL-9, IL-10, IL-11, IL-12, IL-13, IL-14, and IL-15;

the interleukin receptor is selected from the group consisting of IL-1R, IL-2R, IL-3R, IL-4R, IL-5R, IL-6R, IL-7R, IL-8R, IL-9R, IL-10R, IL-11R,, IL-12R, Il-13R,, IL-14R, and IL-15R;

the chemokine is selected from the group consisting of PF4, RANTES, MIP1$\alpha$, MCP1, NAP-2, Gro$\alpha$, Gro$\beta$, and IL-8;

the growth factor is selected from the group consisting of TNFalpha, TGFbeta, TSH, VEGF/VPF, PTHrP, EGF family, FGF, PDGF family, endothelin, Fibrosin (F$_s$F$_{-1}$), human Laminin, and gastrin releasing peptide (GRP);

the growth factor receptor is selected from the group consisting of TNFalphaR, RGFbetaR, TSHR, VEGFR/VPFR, FGFR, EGFR, PTHrPR, PDGFR family , EPO-R, GCSF-R and other hematopoietic receptors;

the interferon receptor is selected from the group consisting of IFNC$\alpha$R, IFN$\beta$R, and IFN$\lambda$R;

the Ig and its receptor is selected from the group consisting of IgE, FceRI, and FCERII;

the tumor antigen is selected from the group consisting of her2-neu, mucin, CEA and endosialin;

the allergen is selected from the group consisting of house dust mite antigen, lol p1 (grass) antigens, and urushiol;

the viral protein is selected from the group consisting of CMV glycoproteins B, H, and gCIII, HIV-1 envelope glycoproteins, RSV envelope glycoproteins, HSV envelope glycoproteins, HPV envelope glycoproteins, Hepatitis family surface antigens;

the toxin is selected from the group consisting of pseudomonas endotoxin and osteopontin/uropontin, snake venom, spider venom, and bee venom conotoxin;

the blood factor is selected form the group consisting of complement C3b, complement C4a, complement C4b-9, Rh factor, fibrinogen, fibrin, and myelin associated growth inhibitor; and

the enzyme is selected from the group consisting of cholesterol ester transfer protein, membrane bound matrix metalloproteases, and glutamic acid decarboxylase (GAD).

21. The immunoglobulin or analog of item 14 wherein said desired antigen is selected from the group consisting of human IL-6, human IL-8, human TNF$\alpha$, human CD4, human L-selectin, human gp39, human IgE, human $\alpha V\beta 3$, human Fibrosin (F$_s$F$_{-1}$), human laminin, human PTHrp, and tetanus toxin C(TTC).

22. A recombinant DNA molecule comprising a nucleotide sequence that encodes the immunoglobulin or analog of item 19-21.

23. The DNA molecule of item 22 wherein said encoding nucleotide sequence is preferably linked to control sequences capable of effecting its expression.

24. A cell or cell line modified to contain the DNA molecule of item 23.

25. A method to produce an immunoglobulin or analog specific for a desired antigen which method comprises culturing the cell or cell line of item 24 under conditions wherein said nucleotide sequence is expressed to produce said immunoglobulin or analog; and recovering the immunoglobulin or analog.

26. An human antibody or analog thereof which is specifically immunoreactive with an antigen selected from the group consisting of transition state mimics; leukocyte markers; histocompatibility antigens; adhesion molecules; interleukins; interleukin receptors; chemokines; growth factors; growth factor receptors; interferon receptors; Igs and their receptors, tumor antigens; allergens; viral proteins; toxins; blood factors; enzymes; and the miscellaneous antigens ganglioside GD3, ganglioside GB2, LMP1, LMP2, eosinophil major basic protein, eosinophil cationic protein, pANCA, Amadori protein, Type IV collagen, glycated lipids, λ-interferon, A7, P-glycoprotein, Fas (AFO-1) and oxidized-LDL.

27. The antibody or analog of item 26 wherein the leukocyte marker is selected from the group consisting of CD2, CD3, CD4, CD5, CD6, CD7, CD8. CD11a,b,c, CD13, CD14, CD18, CD19, CD20, CD22, CD23, CD27 and its ligand, CD28 and its ligands B7.1, B7.2, B7.3, CD29 and its ligand, CD30 and its ligand, CD40 and its ligand gp39, CD44, CD45 and isoforms, CDw52 (Campath antigen), CD56, CD58, CD69, CD72, CTLA-4, LFA-1 and TCR;
the histocompatibility antigen is selected from the group consisting of MHC class I or II, the Lewis y antigens, SLex, SLey, Slea, and SLeb;
the adhesion molecule is selected from the group consisting of VLA-1, VLA-2, VLA-3, VLA-4, VLA-5, VLA-6, $\alpha$V$\beta$3, and LFA-1, Mac-1, p150,95, $\alpha_v\beta_1$, gpIIbIIIa, $\alpha_R\beta_3$, $\alpha_6\beta_4$, $\alpha_v\beta_5$, $\alpha_v\beta_6$, and $\alpha_v\beta_7$, L-selectin, P-selectin, and E-selectin and their counterreceptors VCAM-1, ICAM-1, ICAM-2, and LFA-3;
the interleukin is selected from the group consisting of IL-1, IL-2, IL-3, IL-4, IL-5, IL-6, IL-7, IL-8, IL-9, IL-10, IL-11, IL-12, IL-13, IL-14, ad IL-15;
the interleukin receptor is selected from the group consisting of IL-1R, IL-2R, IL-3R, IL-4R, IL-5R, IL-6R, IL-7R, IL-8R, IL-9R, IL-10R, IL-11R, IL-12R, IL-13R, IL-14R, and IL-15R,
the chemokine is selected from the group consisting of PF4, RANTES, MIP1$\alpha$, MCP1, NAP-2, Gro$\alpha$, Gro$\beta$, and IL-8;
the growth factor is selected from the group consisting of TNFalpha, TGFbeta, TSH, VEGF/VPF, PthrP, EGF family, FGF, PDGF family, endothelia, Fibrosin ($F_sF_{-1}$), human Laminin, and gastrin releasing peptide (GRP);
the growth factor receptor is selected from the group consisting of TNFalphaR, RGFbetaR, TSHR, VEGFR/VPFR, FGFR, EGFR, PTHrPR, PDGFR family, EPO-R, GCSF-R and other hematopoietic receptors;
the interferon receptor is selected from the group consisting of IFN$\alpha$R, IFN$\beta$R, and IFN$\gamma$R;
the Ig and its receptor is selected from the group IgE, FceRI, and FCeRII;
tumor antigen is selected from the group her2-neu, mucin, CEA and endosialin;
the allergen is selected from the group consisting of house dust mite antigen, lol p1 (grass) antigens, and urushiol;
the viral protein is selected from the group consisting of CVM glycoproteins B, H, and GCIII, HIV-1 envelope glycoproteins, RSV envelope glycoproteins, HSV envelope glycoproteins, EBV envelope glycoproteins, VZV envelope glycoproteins, HPV envelope glycoproteins, Hepatitis family surface antigens;
the toxin is selected form the group consisting of pseudomonas endotoxin and osteopontin/uropontin, snake venom, and bee venom;
the blood factor is selected from the group consisting of complement C3b, complement C5a, complement C5b-9, RH factor, fibrinogen, fibrin, and myelin associated growth inhibitor; and
The enzyme is selected from the group consisting of cholesterol ester transfer protein, membrane bound matrix metalloproteases, and glutamic acid decarboxylase (GAD)

28. The antibody or analog of item 26 wherein the desired antigen is selected from the group consisting of human IL-6, human IL-8, human TNF$\alpha$, human CD4, human L-selectin, human gp39, human IgE and tetanus toxin C(TTC).

29. The antibody or analog of item 19 wherein the desired antigen is human IL-6.

30. The antibody or analog of item 19 wherein the described antigen is human IL-8.

31. The antibody or analog of item 19 wherein the desired antigen is human TNF$\alpha$.

32. The antibody or analog of item 19 wherein the desired antigen is human CD4.

33. The antibody or analog of item 19 wherein the desired antigen is human L-selectin.

34. The antibody or analog of item 19 wherein the desired antigen is human gp39.

35. The antibody or analog of item 19 wherein the desired antigen is tetanus toxin C (TTC).

36. The antibody or analog of item 19 wherein the desired antigen is human IgE.

37. The antibody or analog of item 19 wherein the desired antigen is human $\alpha V\beta 3$.

38. The antibody or analog of item 19 wherein the desired antigen is human fibrosin.

39. The antibody or analog of item 19 wherein the desired antigen is human PTHrp.

40. The antibody or analog of item 26 which is an agonist or is a catalyst.

41. A recombinant DNA molecule encoding the antibody of any of item 26-40.

42. A recombinant DNA molecule which comprises an expression system for the antibody or analog of any items 26-40 which expression system comprises a nucleotide sequence encoding said antibody or analog operably linked to control sequences capable of effecting its expression.

43. A recombinant host cell which is modified to contain the DNA molecule of item 42.

44. A method to produce an antibody or analog which method comprises culturing cells of item 43 under conditions wherein said coding sequence is expressed; and recovery the antibody of analog produced.

**Claims**

1. A transgenic mouse wherein all of the somatic and germ cells comprise a DNA fragment of human chromosome 14 from the five most proximal $V_H$ gene segments, continuing through the D segment genes, the J segment genes and the constant region genes through $C\delta$ of the human immunoglobulin heavy chain locus, and wherein said fragment does not contain a $C\gamma$ gene, and wherein said fragment is operably linked to a human $C\gamma 2$ region gene; said transgenic mouse producing fully human IgG2 heavy chains specific for a desired antigen when immunized with said desired antigen.

2. The transgenic mouse according to claim 1, wherein all of the somatic and germ cells of said transgenic mouse further comprise a fragment of human chromosome 2 comprising $V\kappa$, $J\kappa$ and $C\kappa$ gene segments of an immunoglobulin kappa light chain locus, said transgenic mouse producing fully human IgG2 antibodies specific for a desired antigen when immunized with said desired antigen.

3. The transgenic mouse according to claim 1, wherein all of the somatic and germ cells comprise the human DNA contained in the yH1C YAC having ATCC accession no. 74367.

4. The transgenic mouse according to claim 2, wherein all of the somatic and germ cells comprise the human DNA contained in the yH1C YAC having ATCC accession no. 74367.

5. The transgenic mouse according to claim 2, wherein said fragment of human chromosome 2 extends from the three most proximal $V\kappa$ gene segments, continuing through the $J\kappa$ and $C\kappa$ gene segments, through the human kappa deleting element.

6. The transgenic mouse according to claim 4, wherein said fragment of human chromosome 2 extends from the three most proximal $V\kappa$ gene segments, continuing through the $J\kappa$ and $C\kappa$ gene segments, through the human kappa deleting element.

7. The transgenic mouse and progeny according to any one of claims 1-6, wherein all of the somatic and germ cells further comprise:

   (a) inactivated endogenous immunoglobulin heavy chain loci in which all of the J segment genes are deleted to prevent rearrangement and to prevent formation of a transcript of a rearranged locus and the expression of an endogenous immunoglobulin heavy chain; and
   (b) inactivated endogenous immunoglobulin light chain loci in which the $C\kappa$ gene is deleted to prevent expression of an endogenous immunoglobulin light chain;

   wherein said transgenic mouse and progeny lack expression of endogenous immunoglobulin heavy chains.

8. A method for producing a fully human IgG antibody specific for a desired antigen, comprising:

   (a) immunizing a transgenic mouse according to any one of claims 1-7 with said desired antigen; and
   (b) recovering the antibody.

9. The method according to claim 8, wherein the desired antigen is selected from the group consisting of: leukocyte markers; histocompatibility antigens; integrins; adhesion molecules; interleukins; interleukin receptors; chemokines; growth factors; growth factor receptors; interferon receptors; immunoglobulins and their receptors; tumor antigens; allergens; viral proteins; toxins; blood factors; enzymes; ganglioside GD3, ganglioside GM2; LMP1, LMP2; eosinophil major basic protein, eosinophil cationic protein; pANCA; Amadori protein; Type IV collagen; glycated lipids; $\gamma$-interferon; A7; P-glycoprotein; Fas (AFO-1) and oxidized-LDL.

10. The method according to claim 8, wherein the desired antigen is human IL-8.

11. The method according to claim 8, wherein the desired antigen is PTHrp.

12. The yH1C YAC having ATCC accession number 74367.

FIG.1

FIG.2

FIG.3

FIG.4

FIG.5

FIG.6

FIG.7

EP 1 978 033 A2

FIG.8

EP 1 978 033 A2

CMV PROMOTOR

176 NdeI
281 SnaBI
507 EclI36I
507 SacI
595 SacII
593 NalI

Spel 6767

h gp 39 cDNA

1170 BclI
1278 BstEII

puc 19

BsaI 5307

pIKI.1Hu gp39/IRES NEO copy

+

6825 BASE PAIRS
UNIQUE SITES

1557 XcmI
1612 PaeR7I
1612 XhoI

EMCV IRES

ScaI 4896

1940 PmlI
2067 Asp718
2067 KpnI
2073PlMI

Amp$^R$

2348 BbeI
2348 EheI
2348 KasI
2348 NarI
2464 Tth111I

StuI 3777
SfiI 3726
Hpal 3503   3030 BstBI
2864 RsrII

FIG.9

EP 1 978 033 A2

31

FIG.10

FIG.11

```
                              CDR1
                        ┌─────────────────┐
Germline  VH6       AGACCCTCTC ACTCACCTGT GCCATCTCCG GGACAGTGT  CTCTAGCAAC  50
Hybridoma D5. 1.4   AGACCCTCTC ACTCACCTGT GCCATCTCCG GGACAGTGT  CTCTAGCGAC  50
Germline  JH4       ────────── ────────── ────────── ────────── ──────────
Germline  D(N1)     ────────── ────────── ────────── ────────── ──────────
Germline  hMu       ◄───────── ────────── ────────── ─────────── VH6 ─────

                                        CDR2
                        ┌───────────────────────────────────────┐
Germline  VH6       AGTGCTGCTT GGAACTGGAT CAGGCAGTCC CCATCGAGAG GCCTTGAGTG  100
Hybridoma D5. 1.4   AGTGCTGCTT GGAACTGGAT CAGGCAGTCC CCATCGAGAG GCCTTGAGTG  100
Germline  JH4       ────────── ────────── ────────── ────────── ──────────
Germline  D(N1)     ────────── ────────── ────────── ────────── ──────────
Germline  hMu       ────────── ────────── ────────── ────────── ──────────
                    ─────────────────── VH6 ───────────────────────────►

Germline  VH6       GCTGGGAAGG ACATACTACA GGTCCAAGTG GTATAATGAT TATGCAGTAT  150
Hybridoma D5. 1.4   GCTGGGAAGG ACATACTACA GGTCCAAGTG GTATAATGAT TATGCAGTTT  150
Germline  JH4       ────────── ────────── ────────── ────────── ──────────
Germline  D(N1)     ────────── ────────── ────────── ────────── ──────────
Germline  hMu       ────────── ────────── ────────── ────────── ──────────
                    ─────────────────── VH6 ───────────────────────────►

Germline  VH6       CTGTGAAAAG TCGAATAACC ATCAACCCAG ACACATCCAA GAACCAGTTC  200
Hybridoma D5. 1.4   CTGTGAAAAG TCGAATAACC ATCAACCCAG ACACATCCAA GAACCAGTTC  200
Germline  JH4       ────────── ────────── ────────── ────────── ──────────
Germline  D(N1)     ────────── ────────── ────────── ────────── ──────────
Germline  hMu       ────────── ────────── ────────── ────────── ──────────
                    ─────────────────── VH6 ───────────────────────────►

Germline  VH6       TCCCTGCAGC TGAACTCTGT GACTCCCGAG GACACGGCTG TGTATTACTG  250
Hybridoma D5. 1.4   TCCCTGCAGC TGAACTCTGT GACTCCCGAG GACACGGCTG TGTATTACTG  250
Germline  JH4       ────────── ────────── ────────── ────────── ──────────
Germline  D(N1)     ────────── ────────── ────────── ────────── ──────────
Germline  hMu       ────────── ────────── ────────── ────────── ──────────
                    ─────────────────── VH6 ───────────────────────────►

Germline  VH6       TGCAAGAGA─ ────────── ────────── ────────── ─────────  259
Hybridoma D5. 1.4   TGCAAGAGAT ATAGCAGTGG CTGGCGTCCT CTTTGACTGC TGGGGCCAGG  300
Germline  JH4       ────────── ────────── ────────── CTTGACTAGC TGGGGCCAAG  20
Germline  D(N1)     ─────────T ATAGCAGCAG CTGG────── ────────── ─────────  15
Germline  hMu       ────────── ────────── ────────── ────────── ─────────
                    │── VH6 ──►│ ◄──── DN1 ────│   │◄──── JH4 ─────────
```

<p style="text-align:center">FIG.12A</p>

33

```
Germline  VH6                                                                                    259
Hybridoma D5. 1.4  GAACCCTGGT CACCGTCTCC TCAGGGAGTG CATCCGCCCC AACCCTTTTC 350
Germline  JH4      GAACCCTGGT CACCGTCTCC TCA———————— —————————— —————————— 43
Germline  D(N1)    —————————— —————————— —————————— —————————— —————————— 15
Germline  hMu      —————————— —————————— ——GGGAGTG CATCCGCCCC AACCCTTTTC 27
                   ———————— JH4 ————————►│◄————— hμ ——————

Germline  VH6                                                                                    259
Hybridoma D5. 1.4  CCCCTCGTCT CCTGTGAGAA TTCCCCGTCG GATACGAGCA GCGTGGCCGT 400
Germline  JH4      —————————— —————————— —————————— —————————— —————————— 43
Germline  D(N1)    —————————— —————————— —————————— —————————— —————————— 15
Germline  hMu      CCCCTCGTCT CCTGTGAGAA TTCCCCGTCG GATACGAGCA GCGTGGCCGT 77
                   ——————————————————————— hμ ———————————————————————►
```

# FIG.12B

```
Germline B3        GACATCGTGA TGACCCAGTC TCCAGACTCC CTGGCTGTGT CTCTGGGCGA
Hybridoma D5 1.4   ───────── ───────── ───────── ───────── ─────────
Germline JK3       ───────── ───────── ───────── ───────── ─────────
Germline CK        ───────── ───────── ───────── ───────── ─────────


                                              ┌─────────── CDR1 ──────────────┐
Germline B3        GAGGGCCACC ATCAACTGCA AGTCCAGCCA GAGTGTTTTA TACAGCTCCA
Hybridoma D5 1.4   ───────ACC ATCAAGTGCA AGTCCAGCCA GAGTGTTTTG TACACTTCCA
Germline JK3       ───────── ───────── ───────── ───────── ─────────
Germline CK        ───────── ───────── ───────── ───────── ─────────
                        ◄───────────────── B3 ─────────────────


Germline B3        ACAATAAGAA CTACTTAGCT TGGTACCAGC AGAAACCAGG ACAGCCTCCT
Hybridoma D5 1.4    GCAATAAGAA CTACTTAGCT TGGTACCAGC AGAAACCAGG ACAGCCTCCT
Germline JK3       ───────── ───────── ───────── ───────── ─────────
Germline CK        ───────── ───────── ─────── B3 ───────── ───────►


                                       ┌────────── CDR2 ──────────┐
Germline B3        AAGCTGCTCA TTTACTGGGC ATCTACCCGG GAATCCGGGG TCCCTGACCG
Hybridoma D5 1.4   AAACTACTCA TTTACTGGGC ATCTACCCGG GAATCCGGGG TCCCTGACCG
Germline JK3       ───────── ───────── ───────── ───────── ─────────
Germline CK        ───────── ───────── ─────── B3 ───────── ───────►


Germline B3        ATTCAGTGGC AGCGGGTCTG GGACAGATTT CACTCTCACC ATCAGCAGCC
Hybridoma D5 1.4   ATTCAGTGGC AGCGGGTCTG GGACAGATTT CACTCTCACC ATCGGCAGCC
Germline JK3       ───────── ───────── ───────── ───────── ─────────
Germline CK        ───────── ───────── ─────── B3 ───────── ───────►


Germline B3        TGCAGGCTGA AGATGTGGCA GTTTATTACT GTCAGCAATA TTATAGTACT
Hybridoma D5 1.4   TGCAGGCTGA AGATGTGGCA GTTTATTACT GTCAGCAATA TTATACTATT
Germline JK3       ───────── ───────── ───────── ───────── ─────────
Germline CK        ───────── ───────── ─────── B3 ───────── ───────►


Germline B3        CC──────── ───────── ───────── ───────── ─────────
Hybridoma D5 1.4   CCATTCAATT TCGGCCCTGG GACCAGAGTG GATATCAAAC GAACTGTGGC
Germline JK3       ─ATTCACTT TCGGCCCTGG GACCAAAGTG GATATCAAAC ─────────
Germline CK        ───────── ───────── ───────── ───────── GAACTGTGGC
                   ───►│◄───────────── JK3 ─────────────►│◄─ CK ─
```

## FIG.13A

```
Germline B3      —————————  —————————  —————————  —————————  —————————
Hybridoma D5 1.4 TGCACCATCT  GTCTTCATCT  TCCCGCCATC  TGATGAGCAG  TTGAAATCTG
Germline JK3     —————————  —————————  —————————  —————————  —————————
Germline CK      TGCACCATCT  GTCTTCATCT  TCCCGCCATC  TGATGAGCAG  TTGAAATCTG
                 ————————————————————————— CK —————————————————————————▶


Germline B3      —————————  —————————  —————————  —————————  —————————
Hybridoma D5 1.4 GAACTGCCTC  TGTTGTGTGC  CTGCTGAATA  ACTTCTATCC  CAGAGAGGCC
Germline JK3     —————————  —————————  —————————  —————————  —————————
Germline CK      GAACTGCCTC  TGTTGTGTGC  CTGCTGAATA  ACTTCTATCC  CAGAGAGGCC
                 —————————————————————————— CK —————————————————————————▶


Germline B3      —————————  —————————  —————————  —————————  —————————
Hybridoma D5 1.4 AAAGTACAGT  GGAAGGTGGA  TAACGCCCTC  CAATCGGGTT  GGGGAAAAA
Germline JK3     —————————  —————————  —————————  —————————  —————————
Germline CK      AAAGTACAGT  GGAAGGTGGA  TAACGCCCTC  CAATCGGGT—  —————————
                 —————————————————————————— CK —————————————————————————▶
```

# FIG.13B

OD 492nm

○ hκ ANTI-IL8 REACTIVITY
□ hμ ANTI-IL8 REACTIVITY

△ hκ ANTI-TNFα neg. CONTROL
◇ hμ ANTI-TNFα neg. CONTROL

SERUM DILUTIONS

FIG.14

EP 1 978 033 A2

FIG.15A

FIG.15B

[CCTGTCCCTCACCTGCGCTGTCTATGGTGGGTCCTTCAGTGGTTACTACTGGAGCTGGATCCGCC

AGCCCCCAGGGAAGGGACTGGAGTGGATTGGGGAAATCAATCAAAGTGGAAGCACCAATTACAA

CCCGTCCCTCAAGAGTCGAGTCATCATATCAATAGACACGTCCAAGACCCAGTTCTCCCTGAAGT

TGAGCTCTGTGACCGCCGCGGACACGGCTGTGTATTACTGTGCGAGAGA][GACTCCCC][ATGCT

TTTGATATCTGGGGCCAAGGGACAATGGTCACCGTCTCTTCAG]CCTCCACCAAGGGCCCATCGG

TCTTCCCCCTGGCGCCCTGCTCCAGGAGCACCTCCGAGAGCACAGC(GC)GCCCTGGGCTGCCTG

GTCAAGGACTACTTCC

# FIG. 16A

[CAGTCTCCATCCTCCCTGTCTGCATCTGTAGGCGACAGAGTCACCATCACTTGCCAGGCGAGTC

AGGACATTAGTAAGTTTTTAAGTTGGTTTCAACAGAAACCAGGGAAAGCCCCTAAACTCCTGATC

TACGGTACATCCTATTTGGAAACCGGGGTCCCATCAAGTTTCAGTGGAAGTGGATCTGGGACAGA

TTTTACTCTCACCATCAGCAGCCTGCAGCCTGAAGATGTTGCAACATATTTCTGTAACAGNATG

ATGATCTCCC][ATACACTTTCGGCCCTGGGACCAAAGTGGATATCAAAC]GAACTGTGGCTGCAC

CATCTGTCTTCATCTTCCCGCCATCTGATGAGCAGTTGAAATCTGGAACTGCCTCTGTTGTGTGCC

TGCTGAATAACTTCTATCCCAGAGAGGCCAAAGTACAGTGGAAGGTGGATAACGCCC

# FIG. 16B

[AGGTCCCTGAGACTCTCCTGTGCAGCCTCTGGATTCACCTTCAGTAGCTATGGCATGCACTGGNT

CCGCCAGGCTCCAGGCAAGGGGCTGGAGTGGGTGGCAGAAATATCATATGATGGAAGTAATAAA

TACTATGTAGACTCCGTGAAGGGCCGACTCACCATCTCCAGAGACAATTCCAAGAACACGCTGT

ATCTGCAAATGAACAGCCTGAGAGCTGAGGACACGGCTGTGTATTACTGTGCGAGAGA][CCGAC

TGGGGAT][CTTTGACTACTGGGGCCAGGGAACCCTGGTCACCGTCTCCTCAG]CCTCCACCAAGG

GCCCATCGGTCTTCCCCCTGGCGCCCTGCTCCAGGAGCACCTCCGAGAGCACAGC(GC)GGCCCT

GGGCTGCCTGGTCCAAGGACTACTTCCCCCGAACCGGTGACGGTGTCGTGGAACTCAGGCGCTC

TGACCAG

# FIG. 16C

[CTGACNCAGTCTCCAGACTCCCTGGCTGTGTCTCTGGGCGAGAGGGCCACCATCAACTGCAAGT

CCAGCCAGAGTGTTTTATACATCTCCAACAATAAAACTACTTAGCTTGGTACCAGCAGAAACCA

GGACAGTCTCCTAAACTGCTCATTTACTGGGCATCTACCCGGAAATCCGGGGTCCCTGACCGATT

CAGTGGCAGCGGGTCTGGGACAGATTTCACTCTCACCATCAGCAGCCTGCAGGCTGAAGATGTG

GCAGTTTATTACTGTCAACAGTATTATGATACTCC][ATTCACTTTCGGCCCTGGGACCAAAGTGG

ATATCAAAC]GAACTGTGGCTGCACCATCTGTCTTCATCTTCCCGCCATCTGATGAGCAGTTGAAA

TCTGGAACTGCCTCTGTTGTGTGCCTGCTGAATAACTTCTATCCCAGAGAGGCCAAAGTACAGTG

GAAGGTGGNTAACGCCCCA

# FIG. 16D

[TCCCTCACCTGCGCTGTCTATGGTGGGTCCTTCAGTGGTTACTACTGGACCTGGATCCGCCAGCC

CCCAGGGAAGGGGCTGGAGTGGATTGGGGAAATCATTCATCATGGAAACACCAACTACAACCCG

TCCCTCAAGAGTCGAGTCTCCATATCAGTTGACACGTCCAAGAACCAGTTCTCCCTGACACTGAG

CTCTGTGACCGCCGCGGACACGGCTGTGTATTACTGTGCGAGAGG][GGGAGCAGTGGCTGCG][T

TTGACTACTGGGGCCAGGGAACCCTGGTCACCGTCTCCTCAG]CCTCCACCAAGGGCCCATCGGT

CTTCCCCCTGGCGCCCTGCTCCAGGAGCACCTCCGAGAGCACAGC(GC)GGCCCTGGGCTGCCTG

GTCAAGGACTACTTCCCCCGAACCGGTGACGGTGTCGTGGAACTCAGGCGCTCTGACCAGCGGC

GTGCACACCTTCCCA

## FIG. 16E

[TGACCCAGTCTCCATCCTCCCTGTCTGCATCTGTAGGAGACAGAGTCACCATCACTTGCCAGGC

GAGTCAGGACATTAGTAACTATTTAAATTGGTATCAACAGAAAGCAGGGAAAGCCCCTAAGGTCC

TGATCTACGCTGCATCCAATTTGGAAGCAGGGGGTCCCATCAAGGTTCAGTGGAAGTGGATCTGGG

ACAGATTTTACTTTCACCATCAGCAGCCTGCAGCCTGAAGATATTGCAACATATTATTGTCAACA

CTATGATAATCT]A[CTCACTTTCGGCGGAGGGACCAAGGTAGAGATCAAAC]GAACTGTGGCTGC

ACCATCTGTCTTCATCTTCCCGCCATCTGATGAGCAGTTGAAATCTGGACTGCCTCTGTTGTGTG

CCTGCTGAATAACTTCTATCCCAGAGAGGCCAAAGTACAGTGGAAGGTGG

## FIG. 16F

AGTCTCTGAAGATCTCCTGTAAGGGGTTCTGGATACAGCTTTACCAGCTACTGGATCGGCTGGGTG

CGCCAGATGCCCGGGAAAGGCCTGGAGTGGATGGGGGATCATCTATCCTGGTGACTCTGATACCA

GATACAGCCCGTCCTTCCAAGGCCAGGTCACCATCTCAGCCGACAAGTCCATCAGCACCGCCTA

CCTGCAGTGGAGCAGCCTGAAGGCCTCGGACACCGCCATGTATTACTGTGCGAGACA][GGACGG

TG][ACTCCTTTGACTACTGGGGCCAGGGAACCCTGGTCACCGTCTCCTCAG]CCTCCACCAAGGG

CCCATCGGTCTTCCCCCTGGCGCCCTGCTCCAGGAGCACCTCCGAGAGCACAGC(GC)GGCCCTG

GGCTGCCTGGTCCAAGGACTACTTCCCCCGAACCGGTGACGGTGTCGTGGAACTCAGGCGCTCT

GACCAGCGGCGTGCACACCTTCCCACTGCCA

# FIG. 16G

TGTCTGCATCTATTGGAGACAGAGTCACCATCACTTGCCGGGCAAGTCAGAGCATTAGCAACTA

TTTAAATTGGTATCAGCAGAAACCAGGGCAAAGCCCCTAAGTTCCTGATCTATGGTGCATCCAGT

TTGGAAAGTGGGGTCCCATCANGGTTCAGTGGCAGTGGATCTGGGACAGATTTCACTCTCACCAT

CAGCAGCCTGCAACCTGNGGATTTTGCAACTTACTACTGTCAACAGAGTTACAGTAACCC]T[CTC

ACTTTCGGCGGNGGGACCAANGTGGAGATCAAAC]GAACTGTGGCTGCACCATCTGTCTTCATCT

TCCCGCCATCTGATGAGCAGTTGAAATCTGGAACTGCCTCTGTTGTGTGCCTGCTGAATAACTTCT

ATCCCAGAGAGGCCAAAGTACA

# FIG. 16H

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9402602 A

**Non-patent literature cited in the description**

- **GREEN et al.** *Nature Genetics,* 1994, vol. 7, 13-21
- **CALBERTSEN et al.** *PNAS,* 1990, vol. 87, 4256
- **MENDEZ et al.** *Genomics,* 1995, vol. 26, 294-307
- **BURKE et al.** *Science,* 1987, vol. 236, 806-812
- **BROWNSTEIN et al.** *Science,* 1989, vol. 244, 1348-1351
- **BURKE et al.** *Methods in Enzymology,* 1991, vol. 194, 251-270
- **HUSTON et al.** *Methods in Enzymology,* 1991, vol. 203, 46-63
- **TRAUNECKER, A. et al.** *Int. J. Cancer Supp,* 1992, vol. 7, 51-52
- **HIGGINS, P.J.** *J.Infect Disease,* 1992, vol. 166, 198-202
- **FANGER, M.W. et al.** *Cancer Treat Res,* 1993, vol. 68, 181-194
- **FANGER, M.W. et al.** *Crit Rev Immunol,* 1992, vol. 12, 101-124
- **BYERS, B.S. et al.** *Seminars Cell Biol,* 1991, vol. 2, 59-70
- **FANGER, M.W. et al.** *Immunol Today,* 1991, vol. 12, 51-54
- **KOHLER ; MILSTEIN.** *Nature,* 1975, vol. 245, 495
- **GRIFFITHS, A.D. et al.** *EMBO J,* 1994, vol. 13, 3245-3260
- **NISSIM, A. et al.** *EMBO J,* 692-698
- **GRIFFITHS, A.D. et al.** *EMBO J,* vol. 12, 725-734
- **BURTON, D.R. et al.** *Proc. Natl. Acad. Sci. USA,* 1991, vol. 88, 10134-10137
- **ZEBEDEE, S.L. et al.** *Proc. Natl. Acad. Sci. USA,* 1992, vol. 89, 3175-3179
- **BARBAS III, C.F. et al.** *Proc. Natl. Acad. Sci. USA,* 1991, vol. 89, 10164-20168
- **BARBAS III C.F. et al.** *Proc. Natl. Acad. Sci. USA,* 1991, vol. 89, 4457-4461
- **CRAMERI, A. et al.** *BioTechniques,* 1995, vol. 88, 194-196
- **RAPOPORT, B. et al.** *Immunol. Today,* 1995, vol. 16, 43-49
- **PORTOLANO, S. et al.** *J. Immunol.,* 1993, vol. 151, 2839-2851
- **VOGEL, M. et al.** *Eur J. Immunol.,* 1994, vol. 24, 1200-1207
- **GRIFFITHS, A.D. et al.** *EMBO J.,* 1994, vol. 13, 3245-3260
- **GRIFFITHS, A.D. et al.** *EMBO J.,* 1993, vol. 12, 725-734
- **PERSSON, M.A.A. et al.** *Proc. Natl. Acad. Sci. USA,* 1991, vol. 88, 2432-2436
- **GRIFFITHS, A.D.** *Curr. Opin. Immunol.,* 1993, vol. 5, 263-267
- **HOOGENBOOM, H.R. et al.** *J. Mol. Biol.,* 1992, vol. 227, 381-388
- **LERNER, R.A. et al.** *Science,* 1992, vol. 258, 1313-1314
- **NISSIM A. et al.** *EMBO J.,* 1994, vol. 13, 692-698
- **MARKS, J.D. et al.** *J. Mol. Biol.,* 1991, 581-596
- **WINTER, G. et al.** *Annu. Rev. Immunol.,* 1994, vol. 12, 433-55
- **BARBAS III, C.F. et al.** *Proc. Natl. Acad. Sci. USA,* 1991, vol. 88, 7978-7982
- **ROBERTS et al.** *Blood,* 1994, vol. 84, 2878
- **CALLAN, M. et al.** *Proc Natl Acad Sci USA,* 1993, vol. 90, 10454
- **FINER et al.** *Blood,* 1994, vol. 83, 43
- **TEDDER et al.** *J. Immunol,* 1990, vol. 144, 532
- **SUBRANANI et al.** *Mol Cell Biol,* 1981
- **GALFRE, G. ; MILSTEIN, C.** *Methods in Enzymology,* 1981, vol. 73, 3-46
- **RATCLIFFE et al.** *J. Immunol. Methods,* 1990, vol. 127, 109
- **GALFRE G et al.** *Methods in Enzymology,* 1981, vol. 73, 3-46
- **KARLSSON et al.** Kinetic analysis of monoclonal antibody-antigen interaction with a new biosensor based analytical system. *J. Immunol. Methods,* vol. 145, 229

- **MARKS.** *Euro J. Immunol,* 1991, vol. 21, 985-991

- **TOMLINSON et al.** V BASE sequence directory. *MRC Centre for Protein Engineering*